# EUROPÄISCHE PATENTSCHRIFT

(11) **EP 1 713 759 B1**
(45) Veröffentlichungstag und Bekanntmachung des Hinweises auf die Patenterteilung: **03.11.2010**
(21) Anmeldenummer: 05701177.7
(22) Anmeldetag: 26.01.2005
(51) Int. Cl.: C07C 253/10, C07C 253/30

(54) **VERFAHREN ZUR HERSTELLUNG VON ADIPODINITRIL DURCH HYDROCYANIERUNG VON 1,3-BUTADIEN**
METHOD FOR THE PRODUCTION OF ADIPODINITRILE BY HYDROCYANATION OF 1,3-BUTADIENE
PROCEDE DE PRODUCTION D'ADIPODINITRILE PAR HYDROCYANURATION DE 1,3-BUTADIENE

(30) Priorität: 29.01.2004 DE 102004004682
(43) Veröffentlichungstag der Anmeldung: 25.10.2006
(73) Patentinhaber: BASF SE, 67056 Ludwigshafen (DE)
(72) Erfinder: BARTSCH, Michael, 67433 Neustadt (DE); BAUMANN, Robert, 68159 Mannheim (DE); HADERLEIN, Gerd, 67269 Grünstadt (DE); FLORES, Miguel, Angel, E-28300 Aranjuez (Madrid) (ES); JUNGKAMP, Tim, B-2950 Kapellen (BE); LUYKEN, Hermann, 67069 Ludwigshafen (DE); SCHEIDEL, Jens, 69493 Hirschberg (DE); SIEGEL, Wolfgang, 67117 Limburgerhof (DE)
(86) Internationale Anmeldenummer: PCT/EP2005/000717
(87) Internationale Veröffentlichungsnummer: WO 2005/073167

(56) Entgegenhaltungen:
- WO-A-02/30854
- WO-A-02/053527
- WO-A-03/011457
- DE-A1- 10 046 025
- FR-A- 2 830 530
- US-A- 3 496 215
- US-A- 3 850 973
- US-A1- 2001 014 647
- US-B1- 6 169 198
- US-B1- 6 242 633
- MONTGOMERY, J.: "Science of Synthesis" 2001, GOERG THIEME VERLAG , STUTTGART 1 , XP009048800 Seite 11 - Seite 62, Absätze 1.1.1,1.1.1.6,1.1.4.5
- W. C. SEIDEL, C. A. TOLMAN: ANNALS OF THE NEW YORK ACADEMY OF SCIENCES, Bd. 415, 1983, Seiten 201-211, XP009048790
- TOLMAN C A ET AL: "CATALYTIC HYDROCYANATION OF OLEFINS BY NICKEL(O) PHOSPHITE COMPLEXES-EFFECTS OF LEWIS ACIDS" ORGANOMETALLICS, WASHINGTON, DC, US, Bd. 3, 1984, Seiten 33-38, XP001005579 ISSN: 0276-7333

## Beschreibung

Die vorliegende Erfindung betrifft ein Verfahren zur Herstellung von Adipodinitril durch Hydrocyanierung von 1,3-Butadien an einem Katalysator.

Adipodintril ist ein wichtiges Ausgangsprodukt in der Nylonherstellung, das durch zweifache Hydrocyanierung von 1,3-Butadien erhalten wird. Dabei wird in einer ersten Hydrocyanierung 1,3-Butadien zu 3-Pentennitril hydrocyaniert, wobei als Nebenprodukte hauptsächlich 2-Methyl-3-butennitril, 2-Pentennitril, C₉-Nitrile und Methylglutamitril erhalten werden. In einer zweiten, sich anschließenden Hydrocyanierung wird 3-Pentennitril mit Cyanwasserstoff zu Adipodinitril umgesetzt. Beide Hydrocyanierungen werden durch Nickel(0)-Phosphor-Komplexe katalysiert. Darüber hinaus wird in der zweiten Hydrocyanierung von 3-Pentennitril unter Zusatz einer Lewis-Säure, beispielsweise ZnCl₂, FeCl₂, Et₂AlCl, Et₃Al₂Cl₃ oder EtAlCl₂, gearbeitet.

Verfahren zur Herstellung von Adipodimit sind offenbart in WO-03/011457, US-A-6 242 633, US-A-2001/014 647, FR-A-2830530, DE-A-10046025 und US-A349645.

Die bekannten Verfahren zur Herstellung von Adipodinitril führen die in den einzelnen Hydrocyanierungen verwendeten Katalysatoren im Allgemeinen in die jeweiligen Hydrocyanierungen zurück. Die bekannten Verfahren zur Herstellung von Adipodinitril durch Hydrocyanierung von 1,3-Butadien und anschließende Hydrocyanierung des resultierenden 3-Pentennitrils zeigen allerdings keine Zusammenschaltung der in den beiden Hydrocyanierungen verwendeten Katalysatorenkreisläufe auf. Aufgrund von prozessökonomischen Gründen ist dieses aber vorteilhaft.

Demgemäß ist die Aufgabe der vorliegenden Erfindung, ein integriertes Verfahren zur Herstellung von Adipodinitril durch zweifache Hydrocyanierung von 1,3-Butadien bereit zu stellen, das eine Zusammenschaltung der Katalysatorkreisläufe der beiden Hydrocyanierungen während der Herstellung von Adipodinitril ermöglicht.

Die Lösung dieser Aufgabe geht aus von einem Verfahren zur Herstellung von Adipodinitril durch Hydrocyanierung von 1,3-Butadien an einem Katalysator, wobei in einem ersten Verfahrensschritt 1,3-Butadien zu 3-Pentennitril an mindestens einem Nickel(0)-Katalysator hydrocyaniert wird und in einem zweiten Verfahrensschritt 3-Pentennitril zu Adipodinitril an mindestens einem Nickel(0)-Katalysator unter Zugabe mindestens einer Lewis-Säure hydrocyaniert wird, dadurch gekennzeichnet, dass zumindest einer der in den jeweiligen Verfahrens-schritten
(a) Hydrocyanierung von 1,3-Butadien an mindestens einem Nickel(0)-Komplex als Katalysator, wobei ein Hydrocyanierungsstrom 1 resultiert, der 3-Pentennitril, 2-Pentennitril, 2-Methyl-2-butennitril, C₉-Nitrile, 2-Methyl-3-butennitril, den mindestens einen Nickel(0)-Katalysator, Methylglutarnitril, nicht umgesetztes 1,3-Butadien und Reste nicht umgesetzten Cyanwasserstoffs enthält,
(b) Abtrennung des mindestens einen Nickel(0)-Katalysators aus dem Hydrocyanierungsstrom 1 unter Erhalt eines Katalysatorstromes 1, der den mindestens einen Nickel(0)-Katalysator enthält, und eines Hydrocyanierungsstroms 2, der 3-Pentennitril, 2-Pentennitril, 2-Methyl-2-butennitril, 2-Methyl-3-butennitril und C₉-Nitrile enthält,
(c) Regeneration des mindestens einen Nickel(0)-Katalysators in dem Katalysator-strom 1 durch reduktive Nickel-Katalysatorsynthese unter Zugabe von frischem Ligand unter Erhalt eines Katalysatorstroms 2,
(d) Hydrocyanierung von 3-Pentennitril an mindestens einem Nickel(0)-Katalysator und in Gegenwart mindestens einer Lewis-Säure, wobei der Nickel(0)-Katalysator und die Lewis-Säure zumindest teilweise aus dem Katalysatorstrom 2 stammen und ein Hydrocyanierungsstrom 3 resultiert, der den mindestens einen Nickel(0)-Katalysator, Adipodinitril und die mindestens eine Lewis-Säure enthält,
(e) Abtrennung des mindestens einen Nickel(0)-Katalysators aus dem Hydrocyanierungsstrom 3 durch Extraktion mit einem organischen Lösemittel unter Erhalt eines Katalysatorstroms 3, der den mindestens einen Nickel(0)-Katalysator enthält, und eines Produktstromes, der Adipodinitril enthält, wobei der Katalysatorstrom 3 zumindest teilweise in Verfahrensschritt (a) zurückgeführt werden kann.

Das erfindungsgemäße Verfahren ist dann dadurch gekennzeichnet, dass zumindest einer der in den jeweiligen Verfahrensschritten verwendeten Nickel(0)-Katalysatoren zumindest teilweise in den anderen Verfahrensschritt überführt wird.

Durch die Zusammenschaltung der zuvor beschriebenen Katalysatorströme ist ein wirtschaftliches Verfahren zur Herstellung von Adipodinitril möglich.

Unter 2-Pentennitril wird im Sinne der vorliegenden Erfindung auch Isomere von 2-Pentennitril verstanden.

Unter 2-Methyl-2-butennitril wird im Sinne der vorliegenden Erfindung auch Isomere von 2-Methyl-2-butennitril verstanden.

Der ***Verfahrensschritt (a)*** umfasst die Umsetzung von 1,3-Butadien und Cyanwasserstoff an mindestens einem Katalysator. Als Katalysator wird vorzugsweise ein homogen gelöster Katalysator verwendet. Besonders bevorzugt werden homogen gelöste Nickel(0)-Katalysatoren verwendet.

Die besonders bevorzugt verwendeten Nickel(0)-Katalysator-Komplexe sind vorzugsweise mit Phosphorliganden stabilisiert.

Bei den Ni(0)-Komplexen, die phosphorhaltige Liganden und/oder freie phosphorhaltige Liganden enthalten, handelt es sich bevorzugt um homogen gelöste Nickel(0)-Komplexe.

Die phosphorhaltigen Liganden der Nickel(0)-Komplexe und die freien phosphorhalti-gen Ligandensind vorzugsweise ausgewählt aus mono- oder bidentaten Phosphinen Phosphiten, Phosphiniten und Phosphoniten.

Diese phosphorhaltigen Liganden weisen vorzugsweise die Formel I auf:

P (X¹R¹) (X²R²) (X³R³) (I)

Unter Verbindung I wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff oder Einzelbindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauerstoff, so stellt Verbindung I ein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so dass Verbindung I vorteilhaft ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl, Ethyl, n-Propyl, i-Propyl, n-Butyl, 1-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³ o-Tolyl-Gruppen sein.

Als besonders bevorzugte Verbindungen I können solche der Formel I a

(o-Tolyl-O-)_{w} (m-Tolyl-O-)ₓ (p-Tolyl-o-)_{y} (Phenyl-O-)_{z} P (Ia)

eingesetzt werden, wobei w, x, y und z eine natürliche Zahl bedeuten, und folgende Bedingungen gelten: w + x + y + z = 3 und w, z ≤ 2.

Solche Verbindungen I a sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P, (m-Tolyl-O-)₂(Phenyl-O-)P, (o-Tolyl-O-)₂(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen.

Gemische enthaltend (m-Tolyl-O-)₃P, (m -Tolyl-O-)₂(p-Tolyl-O-)P, (m-Tolyl-O-)(p-Tolyl-O-)₂P und (p-Tolyl-O-)₃P kann man beispielsweise durch Umsetzung eines Gemisches enthaltend m-Kresol und p-Kresol, insbesondere im Molverhältnis 2 : 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel I b in Betracht:

P (O-R¹)ₓ (O-R²)_{y} (O-R³)_{z} (O-R⁴)ₚ (Ib)

mit
- R¹:: aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromati- schen System verbindet, anellierten aromatischen System,
- R²:: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoff- atom, das das Phosphoratom mit dem aromatischen System verbindet, oder mit einem in m-Stellung zu dem Sauerstoffatom, das das Phosphoratom mit dem aromatischen System verbindet, anellierten aromatischen System, wobei der a- romatische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aro- matische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x :: 1 oder 2,
- y, z, p:: unabhängig voneinander 0, 1 oder 2 mit der Maßgabe, dass x+y+z+p = 3.

Bevorzugte Phosphite der Formel I b sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl-oder 1-Naphthyl- Gruppen in Betracht.

Als Rest R² sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung I b ergeben sich folgende Möglichkeiten:

| x | y | z | p |
|---|---|---|---|
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel I b sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist.

Besonders bevorzugte Phosphite der Formel I b sind solche, in denen R¹ der o-Isopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-Isopropyl-phenyl-Rest, R² der 2-Naphthylrest und R³ der p-Tolylrest ist mit den in der Tabelle genannten Indizes; sowie Gemische dieser Phosphite.

Phosphite der Formel Ib können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I b.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃. Weitere Einzelheiten zu den Reaktionsbedingungen bei der Herstellung der Phosphite I b und zur Aufarbeitung sind der DE-A 199 53 058 zu entnehmen.

Die Phosphite I b können auch in Form eines Gemisches verschiedener Phosphite I b als Ligand verwendet werden. Ein solches Gemisch kann beispielsweise bei der Herstellung der Phosphite I b anfallen.

Es ist allerdings bevorzugt, dass der phosphorhaltige Ligand mehrzähnig, insbesondere zweizähnig ist. Daher weist der verwendete Ligand vorzugsweise die Formel II 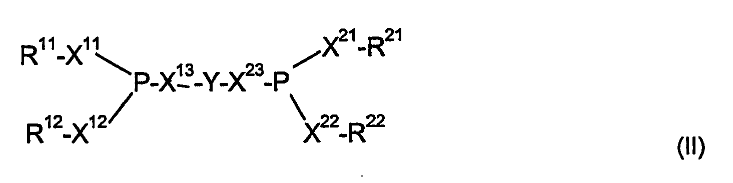 auf, worin bedeuten
- X¹¹, X¹², X¹³, X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹, R²²: unabhängig voneinander gleiche oder unterschiedliche, einzelne oder verbrückte organische Reste,
- Y: Brückengruppe

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen..

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Die beschriebenen Verbindungen I, Ia, Ib und II sowie deren Herstellung sind an sich bekannt. Als phosphorhaltiger Ligand können auch Mischungen, enthaltend mindestens zwei der Verbindungen I, a, Ib und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus Tritolylphosphit, bidentaten phosphorhaltigen Chelatliganden, sowie den Phosphiten der Formel Ib

P (O-R¹)ₓ (O-R²)_{y} (O-R³), (O-R⁴)ₚ (Ib)

worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen.

Die Hydrocyanierung kann in Gegenwart oder in Abwesenheit von einem Lösemittel durchgeführt werden. Wenn ein Lösemittel verwendet wird, so sollte das Lösemittel bei der gegebenen Reaktionstemperatur und dem gegebenen Reaktionsdruck flüssig und inert gegenüber den ungesättigten Verbindungen und dem mindestens einen Katalysator sein. Im Allgemeinen werden als Lösemittel Kohlenwasserstoffe, beispielsweise Benzol oder Xylol, oder Nitrile, beispielsweise Acetonitrile oder Benzonitril, verwendet. Vorzugsweise wird allerdings ein Ligand als Lösemittel verwendet. Ferner ist es möglich, mehrere, wie zwei oder drei, Lösemittel zu verwenden.

Die in Verfahrensschritt (a) verwendeten Katalysatoren können beispielsweise durch reduktive Katalysatorsynthese hergestellt werden. Hierzu wird eine Nickel(II)-Quelle mit Ligand nach allgemein bekannten Verfahren, wie beispielsweise in US 6,127,567 und den dort zitierten Referenzen sowie den deutschen Patentanmeldungen DE 103 51 000.1, DE 103 51 002.8 und DE 103 51 003.6 der BASF AG beschrieben, zum Nickel(0)-Komplex umgesetzt.

Der Verfahrensschritt (a) des erfindungsgemäßen Verfahrens kann in jeder geeigneten, dem Fachmann bekannten Vorrichtung durchgeführt werden. Für die Reaktion kommen hierfür übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann in mehreren, wie zwei oder drei, Apparaten durchgeführt werden.

In einer bevorzugten Ausführungsform des erfindungsgemäßen Verfahrens haben sich Reaktoren mit Rückvermischungscharakteristik oder Kaskaden von Reaktoren mit Rückvermischungscharakteristik als vorteilhaft erwiesen. Als besonders vorteilhaft haben sich Kaskaden aus Reaktoren mit Rückvermischungscharakteristik erwiesen, die in Bezug auf die Dosierung von Cyanwasserstoff in Querstromfahrweise betrieben werden.

Die Reaktion kann in Batchfahrweise, kontinuierlich oder im Semibatchbetrieb durchgeführt werden.

Vorzugsweise wird die Hydrocyanierung kontinuierlich in einem oder mehreren gerührten Verfahrensschritten durchgeführt. Wenn eine Mehrzahl von Verfahrensschritten verwendet wird, so ist es bevorzugt, dass die Verfahrensschritte in Serie gestaltet sind. Dabei wird das Produkt von einem Verfahrensschritt direkt in den nächsten Verfahrensschritt überführt. Der Cyanwasserstoff kann direkt in den ersten Verfahrensschritt oder zwischen den einzelnen Verfahrensschritten zugeführt werden.

Wenn das erfindungsgemäße Verfahren im Semibatchbetrieb durchgeführt wird, so ist es bevorzugt, dass im Reaktor die Katalysatorkomponenten und 1,3-Butadien vorgelegt werden, während Cyanwasserstoff über die Reaktionszeit hinweg in die Reaktionsmischung dosiert wird.

Die Hydrocyanierungsreaktion kann durchgeführt werden, in dem die Vorrichtung mit allen Reaktanten bestückt wird. Bevorzugt ist allerdings, wenn die Vorrichtung mit dem mindestens einen Katalysator, 1,3-Butadien und gegebenenfalls dem Lösemittel gefüllt wird. Der gasförmige Cyanwasserstoff schwebt vorzugsweise über der Oberfläche der Reaktionsmischung oder wird vorzugsweise durch die Reaktionsmischung geleitet. Eine weitere Verfahrensweise zum Bestücken der Vorrichtung ist das Befüllen der Vorrichtung mit dem mindestens einen Katalysator, Cyanwasserstoff und gegebenenfalls dem Lösemittel und Zuspeisen des 1,3-Butadiens langsam zu der Reaktionsmischung. Alternativ ist auch möglich, dass die Reaktanten in den Reaktor eingeführt werden und die Reaktionsmischung auf die Reaktionstemperatur gebracht wird, bei der der Cyanwasserstoff flüssig zu der Mischung gegeben wird. Darüber hinaus kann der Cyanwasserstoff auch vor Erwärmen auf Reaktionstemperatur zugegeben werden. Die Reaktion wird unter konventionellen Hydrocyanierungsbedingungen für Temperatur, Atmosphäre, Reaktionszeit, etc. durchgeführt.

Die Reaktion wird vorzugsweise bei Drücken von 0,1 bis 500 MPa, besonders bevorzugt 0,5 bis 50 MPa, insbesondere 1 bis 5 MPa durchgeführt. Die Reaktion wird vorzugsweise bei Temperaturen von 273 bis 473 K, besonders bevorzugt 313 bis 423 K, insbesondere bei 333 bis 393 K durchgeführt. Dabei haben sich durchschnittliche mittlere Verweilzeiten der flüssigen Reaktorphase im Bereich von 0,001 bis 100 Stunden, vorzugsweise 0,05 bis 20 Stunden, besonders bevorzugt 0,1 bis 5 Stunden, pro Reaktor als vorteilhaft erwiesen.

Die Reaktion kann in einer Ausführungsform in flüssiger Phase in Gegenwart einer Gasphase und gegebenenfalls einer festen suspendierten Phase ausgeführt werden. Dabei können die Ausgangsstoffe Cyanwasserstoff und 1,3-Butadien jeweils flüssig oder gasförmig zudosiert werden.

Die Reaktion kann in einer weiteren Ausführungsform in flüssiger Phase durchgeführt werden, wobei der Druck im Reaktor so bemessen ist, dass alle Edukte wie 1,3-Butadien, Cyanwasserstoff und der mindestens eine Katalysator flüssig zudosiert werden und in der Reaktionsmischung in flüssiger Phase vorliegen. Dabei kann eine feste suspendierte Phase im Reaktionsgemisch vorliegen, die auch zusammen mit dem mindestens einen Katalysator zudosiert werden kann, beispielsweise bestehend aus Abbauprodukten des Katalysatorsystems, enthaltend unter anderem Nickel(II)-Verbindungen.

Im Verfahrensschritt (a) wird ein Hydrocyanierungsstrom 1, der 3-Pentennitril, 2-Pentennitril, 2-Methyl-2-butennitril, C₉-Nitrile, 2-Methyl-3-butennitril, den mindestens einen Nickel(0)-Katalysator, Methylglutardintirl nicht umgesetztes 1,3-Butadien und Reste nicht umgesetzten Cyanwasserstoffs enthält, erhalten.. Dieser Hydrocyanierungsstrom 1 enthält vorzugsweise 1 bis 80 Gew.-%, besonders bevorzugt 10 bis 50 Gew.-%, des mindestens einen Katalysators, 0,1 bis 50 Gew.-%, besonders bevorzugt 1 bis 25 Gew.-%, 1,3-Butadien, 1 bis 80 Gew.-%, besonders bevorzugt 10 bis 50 Gew.-%, Pentennitrile, umfassend trans-3-Pentennitril, 2-Methyl-3-butennitril sowie weitere Pentennitrilisomere, 0,1 Gew.-ppm bis 10 Gew.-%, besonders bevorzugt 10 Gew.-ppm bis 1 Gew.-%, Cyanwasserstoff und weniger als 5 Gew.-%, besonders bevorzugt weniger als 4 Gew.-%, Methylglutardinitril. Die Menge an C₉-Nitrilen ist vorzugsweise gering.

Der Hydrocyanierungsstrom 1, der 3-Pentennitril, 2-Methyl-3-butennitril, 2-Pentennitril, C₉-Nitrile, Methylglutamitril und den mindestens einen Nickel(0)-Katalysator enthält, wird anschließend in ***Verfahrensschritt (b)*** überführt.

In diesem Verfahrensschritt (b) erfolgt eine Abtrennung des mindestens einen Nickel(0)-Katalysators aus dem Hydrocyanierungsstrom 1. Dabei wird ein Katalysator-strom 1 erhalten, der den mindestens einen Nickel(0)-Katalysator enthält. Darüber hinaus wird ein Hydrocyanierungsstrom 2 erhalten, der 3-Pentennitril, 2-Pentennitril, 2-Methyl-2-butennitril, C₉-Nitrile 2-Methyl-3-butennitril und Methylglutarnitril enthält. Die Abtrennung des mindestens einen Nickel(0)-Katalysators erfolgt vorzugsweise durch Destillation.

Die Abtrennung des mindestens einen Nickel(0)-Katalysators aus dem Hydrocyanierungsstrom 1 durch Destillation kann in jeder geeigneten, dem Fachmann bekannten Vorrichtung durchgeführt werden. Für die Destillation geeignete sind Vorrichtungen, wie sie beispielsweise in: Kirk-Othmer, Encyclopedia of Chemical Technology, 4. Ed., Vol. 8, John Wiley & Sons, New York, 1996, Seiten 334 bis 338 beschrieben sind, wie Siebbodenkolonnen, Glockenbodenkolonnen, Packungskolonnen, Füllkörperkolonnen, die auch als Trennwandkolonnen betrieben werden können. Diese Destillationsvorrichtungen sind jeweils mit geeigneten Vorrichtungen zur Verdampfung, wie Fallfilmver dampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation kann in mehreren, wie zwei oder drei Vorrichtungen durchgeführt werden. Die Destillation kann zudem einstufig im Sinne einer Teilverdampfung des Zulaufstroms erfolgen. Besonders bevorzugt sind Ausführungsformen bei denen die Sumpftemperaturen der Destillationsvorrichtungen alle kleiner als 140 °C, bevorzugt kleiner 130 °C, besonders bevorzugt kleiner als 120 °C sind. Weitere bevorzugte Ausführungsformen sehen für die Summe der mittleren Verweilzeiten in den Sümpfen der Destillationsvorrichtungen höchstens 10 Stunden, besonders bevorzugt höchstens 5 Stunden, insbesondere höchstens 1 Stunde vor. Insbesondere bevorzugt sind Ausführungsformen, welche die beiden zuvor aufgeführten Merkmale gleichzeitig erfüllen. Die Ausführungsformen zeichnen sich durch besonders schonende Katalysatorhandhabung aus.

Der in Verfahrensschritt (b) abgetrennte mindestens eine Nickel(0)-Katalysator in Katalysatorstrom 1 wird dann anschließend in einem ***Verfahrensschritt (c)*** durch reduktive Nickel-Katalysatorsynthese gegebenenfalls unter Zugabe von frischem Ligand und gegebenenfalls unter Zugabe zusätzlicher Lewis-Säure regeneriert.

Die Regeneration des mindestens einen Nickel(0)-Katalysators kann in jeder geeigneten, dem Fachmann bekannten Vorrichtung durchgeführt werden. Geeignet sind beispielsweise hierfür übliche Vorrichtungen, die beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren. Die Reaktion kann auch in mehreren, beispielsweise 2 oder 3 Vorrichtungen, durchgeführt werden;

Eine bevorzugte Ausführungsform der reduktiven Nickel-Katalysatorsynthese ist in der prioritätsälteren, nicht vorveröffentlichten deutschen Patentanmeldung DE 103 51 000.1 mit dem Titel "Verfahren zur Herstellung von Nickel(0)-Phosphorligand-Komplexen" der BASF AG beschrieben. Demgemäß erfolgt die Herstellung des Nickel(0)-Katalysators dadurch, dass man ein Nickel(II)-Ether-Addukt in Gegenwart mindestens eines Phosphor-haltigen Liganden reduziert. Das hierfür zu verwendende Nickel(II)-Ether-Addukt wird vorzugsweise dadurch hergestellt, dass man ein Nickelhalogenid in Wasser löst, mit einem Ether und einem organischen Nitril, gegebenenfalls unter Rühren, versetzt und anschließend Wasser und gegebenenfalls Ether entfernt. Das Nickel(II)-Ether-Addukt ist vorzugsweise wasserfrei und enthält in einer bevorzugten Ausführungsform ein Nickelhalogenid. Als Nickelhalogenid kommen Nickelchlorid, Nickelbromid und Nickeliodid in Frage. Bevorzugt ist Nickelchlorid. Das verwendete Nickel(II)-Ether-Addukt umfasst vorzugsweise einen Sauerstoff-haltigen, Schwefel-haltigen oder gemischt Sauerstoff-Schwefel-haltigen Ether. Dieser ist vorzugsweise ausgewählt aus der Gruppe, bestehend aus Tetrahydrofuran, Dioxan, Diethylether, Din-propylether, Diisopropylether, Di-n-butylether, Di-sec-butylether, Ethylenglykoldialkylether, Diethylenglykoldialkylether und Triethylenglykoldialkylether. Als Ethylenglykoldialkylether wird bevorzugt Ethylenglykoldimethylether (1,2-Dimethoxyethan, Glyme) und Ethylenglykoldiethylether verwendet. Als Diethylenglykoldialkylether wird bevorzugt Diethylenglykoldimethylether (Diglyme) verwendet. Als Triethylenglykoldialkylether wird bevorzugt Triethylenglykoldimethylether (Triglyme) verwendet. Das zur Herstellung des Nickel(0)-Komplexes verwendete Reduktionsmittel ist vorzugsweise ausgewählt aus der Gruppe, bestehend aus Metallen, die elektropositiver als Nickel sind, Metallalkylen, elektrischem Strom, komplexen Hydriden und Wasserstoff.

In einer weiteren Ausführungsform kann der Nickel(0)-Katalysator durch ein Verfahren hergestellt werden, das in der prioritätsälteren, nicht vorveröffentlichten deutschen Patentanmeldung DE 103 51 002.8 mit dem Titel "Verfahren zur Herstellung von Nickel(0)-Phosphorligand-Komplexen" der BASF AG beschrieben ist. Demnach erfolgt die Herstellung des Nickel(0)-Komplexes dadurch, dass eine Nickel(II)-Quelle, die Nickelbromid, Nickeliodid oder Mischungen davon enthält, in Gegenwart mindestens eines Phosphor-haltigen Liganden reduziert wird. Dabei wird die Nickel(II)-Quelle vorzugsweise ohne vorherige besondere Trocknung verwendet. Es ist bevorzugt, dass die Herstellung dabei vorzugsweise in einem Lösemittel erfolgt, das ausgewählt ist aus der Gruppe, bestehend aus organischen Nitrilen, aromatischen oder aliphatischen Kohlenwasserstoffen oder Mischungen davon. Als Reduktionsmittel werden vorzugsweise Metalle, die elektropositiver als Nickel sind, verwendet. Gleichfalls ist auch die Vewendung von Metallalkylen, elektrischem Strom, komplexen Hydriden oder Wasserstoff möglich.

Darüber hinaus kann der in dem erfindungsgemäßen Verfahren verwendete Nickel(0)-Katalysator auch nach einem Verfahren hergestellt werden, das in der prioritätsälteren, nicht vorveröffentlichten deutschen Patentanmeldung DE 103 51 003.6 mit dem Titel "Einsatz von azeotrop-getrocknetem Nickel(II)-Halogeniden" der BASF AG beschrieben ist, hergestellt werden. Demnach erfolgt die Herstellung des Nickel(0)-Komplexes dadurch, dass ein durch Azeotropdestillation getrocknetes, wenn zuvor wasserhaltiges, Nickel(II)-halogenid in Gegenwart mindestens eines Phosphor-haltigen Liganden reduziert wird. Das Nickel(II)-halogenid ist vorzugsweise ausgewählt aus der Gruppe, bestehend aus Nickel(II)-chlorid, Nickel(II)-bromid und Nickel(II)-iodid. Das durch Azeotropdestillation getrocknete Nickel(II)-halogenid wird vorzugsweise durch ein Verfahren zur Entfernung von Wasser aus den entsprechenden wasserhaltigen Nickel(II)-halogeniden hergestellt, wobei die Mischung mit einem Verdünnungsmittel versetzt wird, dessen Siedepunkt im Falle der Nichtazeotropbildung des genannten Verdünnungsmittels mit Wasser unter den Druckbedingungen der nachfolgend genannten Destillation höher ist als der Siedepunkt von Wasser und das an diesem Siedepunkt des Wassers flüssig vorliegt oder das ein Azeotrop oder Heteroazeotrop mit Wasser unter den Druck- und Temperaturbedingungen der nachfolgend genannten Destillation bildet, und die Mischung, enthaltend das wasserhaltige Nickel(II)-halogenid und das Verdünnungsmittel, unter Abtrennung von Wasser oder des genannten Azeotrops oder des genannten Heteroazeotrops von dieser Mischung und unter Erhalt einer wasserfreien Mischung, enthaltend Nickel(II)-halogenid und das besagte Verdünnungsmittel destilliert wird. Diese Mischung kann gegebenenfalls eine Suspension sein. Die Mischung ist charakterisiert durch einen Restgehalt an Wasser kleiner als 1000 Gew.ppm, besonders bevorzugt kleiner als 500 Gew.-ppm, insbesondere kleiner als 100 Gew.-ppm. Das verwendete Verdünnungsmittel ist vorzugsweise ein organisches Verdünnungsmittel mit mindestens einer Nitrilgruppe, vorzugsweise Pentennitril. Die Reduktion zur Herstellung des entsprechenden Nickel(0)-Komplexes erfolgt vorzugsweise durch Metalle, die elektroposifiver als Nickel sind. Alternativ ist auch die Verwendung von Metallalkylen, elektrischem Strom, Metallhydriden und Wasserstoff möglich.

Der in den Verfahren gemäß den zuvor beschriebenen Patentanmeldungen DE 103 51 000.1, DE 103 51 002.8 und DE 103 51 003.6 verwendete Ligand kann auch in einer Ligandlösung vorliegen, die bereits als Katalysatorlösung in Hydrocyanierungsreaktionen eingesetzt wurde und somit an Nickel(0) abgereichert ist.

Im Sinne der vorliegenden Erfindung wird unter einer Lewis-Säure eine einzelne Lewis-Säure, wie auch ein Gemisch aus mehreren, wie 2, 3 oder 4 Lewis-Säuren, verstanden.

Als Lewis-Säure kommen dabei anorganische oder organische Metall-Verbindungen in Betracht, in denen das Kation ausgewählt ist aus der Gruppe bestehend aus Scandium, Titan, Vanadium, Chrom, Mangan, Eisen, Kobalt, Kupfer, Zink, Bor, Aluminium, Yttrium, Zirkonium, Niob, Molybdän, Cadmium, Rhenium und Zinn. Beispiele umfassen ZnBr₂, Znl₂, ZnCl₂, ZnSO₄, CuCl₂, CuCl, Cu(O₃SCF₃)₂, CoCl₂, Col₂, Fel₂, FeCl₃, FeCl2, FeCl₂(THF)₂, TiCl₄(THF)₂, TiCl₄, TiCl₃, ClTi(O-i-Propyl)₃, MnCl₂, ScCl₃, AlCl₃, (C₈H₁₇)AiCl₂, (C₈H₁₇)₂AlCl, (i-C₄H₉)₂AlCl, (C₆H₅)₂AlCl, (C₆H₅)AlCl₂, ReCl₅, ZrCl₄, NbCl5, VCl₃, CrCl₂, MoCl₅, YCl₃, CdCl₂, LaCl₃, Er(O₃SCF₃)₃, Yb(O₂CCF₃)₃, SmCl₃, B(C₈H₅)₃, TaCl₅, wie sie beispielsweise in US 6,127,567, US 6,171,996 und US 6,380,421 beschrieben sind. Weiterhin kommen in Betracht Metallsalze, wie ZnCl₂, Col₂ und SnCl₂ und organometallische Verbindungen, wie RAlCl₂, R₂AlCl, RSnO₃SCF₃ und R₃B, wobei R eine Alkyl- oder Aryl-Gruppe ist, wie sie beispielsweise in US 3,496,217, US 3,496,218 und US 4,774,353 beschrieben sind. Weiterhin können gemäß US 3,773,809 als Promotor ein Metall in kationischer Form, ausgewählt aus der Gruppe bestehend aus Zink, Cadmium, Beryllium, Aluminium, Gallium, Indium, Thallium, Titan, Zirkonium, Hafnium, Erbium, Germanium, Zinn, Vanadium, Niob, Scandium, Chrom, Molybdän, Wolfram, Mangan, Rhenium, Palladium, Thorium, Eisen und Kobalt, vorzugsweise Zink, Cadmium, Titan, Zinn, Chrom, Eisen und Kobalt, eingesetzt werden, wobei der anionische Teil der Verbindung ausgewählt sein kann aus der Gruppe bestehend aus Halogeniden, wie Fluorid, Chlorid, Bromid und Jodid, Anionen niedriger Fettsäuren mit von 2 bis 7 Kohlenstoffatomen, HPO₃₂-, H₃PO₂-, CF₃COO-, C₇H₁₅OSO₂oder SO₄₂-. Weiterhin sind aus US 3,773,809 als geeignete Promotoren Borhydride, Organoborhydride und Borsäureester der Formel R₃B und B(OR)₃, wobei R ausgewählt ist aus der Gruppe bestehend aus Wasserstoff, Aryl-Radikale mit zwischen 6 und 18 Kohlenstoff-Atomen, mit Alkyl-Gruppen mit 1 bis 7 Kohlenstoff-Atomen substituierte Aryl-Radikale und mit Cyano-substituierte Alkyl-Gruppen mit 1 bis 7 KohlenstoffAtomen substituierte Aryl-Radikale, vorteilhaft Triphenylbor, genannt. Weiterhin können, wie in US 4,874,884 beschrieben, synergistisch wirksame Kombinationen von Lewis-Säuren eingesetzt werden, um die Aktivität des Katalysatorsystems zu erhöhen. Geeignete Promotoren können beispielsweise aus der Gruppe bestehend aus CdCl₂, FeCl₂, ZnCl₂, B(C₆H₅)₃ und (C₆H₅)₃SnX, mit X=CF₃SO₃, CH₃C₆H₄SO₃ oder (C₆H₅)₃BCN ausgewählt werden, wobei für das Verhältnis von Promotor zu Nickel ein Bereich von vorzugsweise etwa 1:16 bis etwa 50:1 genannt ist.

Im Sinne der vorliegenden Erfindung umfasst der Begriff Lewis-Säure auch die in US 3,496,217, US 3,496,218, US 4,774,353, US 4,874,884, US 6,127,567, US 6,171,996 und US 6,380,421 genannten Promotoren.

Als besonders bevorzugte Lewis-Säuren kommen unter den genannten insbesondere Metallsalze, besonders bevorzugt Metallhalogenide, wie Fluoride, Chloride, Bromide, Jodide, insbesondere Chloride, in Betracht; von denen wiederum Zinkchlorid, Eisen-(II)-Chlorid und Eisen-(III)-chlorid besonders bevorzugt sind.

In der bevorzugten Ausführungsform des erfindungsgemäßen Verfahrens wird anschließend in einem ***Verfahrensschritt (d)*** 3-Pentennitril an dem einem Nickel(0)-Katalysator und in Gegenwart einer Lewis-Säure, wobei der Nickel(0)-Katalysator und die Lewis-Säure zumindest teilweise aus dem Katalysatorstrom 2 stammen, hydrocyaniert. Dabei wird ein Hydrocyanierungsstrom 3 erhalten. Dieser enthält den mindestens einen Nickel(0)-Katalysator, Adipodintril und die mindestens eine Lewis-Säure.

Vorteilhafte Bedingungen der Hydrocyanierung von 3-Pentennitril können US 6,127,567 und US 5,693,843 entnommen werden, deren Inhalt diesbezüglich durch Bezugnahme in die vorliegende Erfindung eingeschlossen wird.

Der Verfahrensschritt (d) kann in jeder geeigneten, dem Fachmann bekannten Apparatur durchgeführt werden. Für die Reaktion kommen somit übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann in mehreren, wie zwei oder drei, Apparaten durchgeführt werden.

In einer bevorzugten Ausführungsform des erfindungsgemäßen Verfahrens haben sich Reaktoren mit Rückvermischungscharakteristik oder Kaskaden von Reaktoren mit Rückvermischungscharakteristik als vorteilhaft erwiesen. Als besonders vorteilhaft haben sich Kaskaden aus Reaktoren mit Rückvermischungscharakteristik erwiesen, die in Bezug auf die Dosierung von Cyanwasserstoff in Querstromfahrweise betrieben werden.

Gegebenenfalls kann auch nur ein Teil des Katalysatorstroms 2 in der Hydrocyanierung von Verfahrensschritt (d) verwendet werden. Der restliche Teil wird dann als By-pass-Strom 1 vorzugsweise direkt in den Verfahrensschritt (e) gefahren.

In einem sich anschließenden ***Verfahrensschritt (e)*** wird der mindestens eine Nickel(0)-Katalysator aus dem Hydrocyanierungsstrom 3 vorzugsweise abgetrennt. Dieses erfolgt vorzugsweise durch Extraktion mit einem organischen Lösemittel. Dabei wird ein Katalysatorstrom 3, der den mindestens einen Nickel-Katalysator enthält, und ein Produktstrom, der Adipodinitril enthält, erhalten.

Der Verfahrensschritt (e) kann in jeder geeigneten, dem Fachmann bekannten Apparatur durchgeführt werden. Die Extraktion des Verfahrensschritts (e) findet vorzugsweise in Gegenstrom-Extraktionskolonnen, Mixer-Settler-Kaskaden oder Kombinationen von Mixer-Settler-Kaskaden mit Kolonnen statt. Besonders bevorzugt ist die Verwendung von Gegenstrom-Extraktionskolonnen, die insbesondere mit Blechpackungen als dispergierende Elemente ausgestattet sind. Dieses ist überraschend, da der Hydrocyanierungsaustrag feststoffbeladen ist. Erfindungsgemäß wurde festgestellt, dass der Nickel(II)cyanid-haltige Feststoff, der in den Hydrocyanierungsschritten (a) und/oder (d) entsteht, entgegen den Erwartungen unter den Bedingungen in der Extraktion nicht zum Aufwachsen neigt und keine merklichen Ablagerungen auf Kolonneneinbauten bildet.

In einer weiteren besonders bevorzugten Ausführungsform wird die Gegenstromextraktion in einer kompartimentierten, gerührten Extraktionskolonne ausgeführt.

In einer bevorzugten Ausführungsform wird das Extraktionsmittel als disperse Phase eingesetzt und der Hydrocyanierungsstrom 3 als kontinuierliche Phase eingesetzt.

In der Extraktion kann ein Phasenverhältnis von 0,1 bis 10, berechnet als Verhältnis von Volumen des zugeführten Extraktionsmittels zu Volumen der zu extrahierenden Mischung, eingesetzt werden. In einer bevorzugten Ausführungsform wird die Extraktion mit einem Phasenverhältnis von 0,4 bis 2,5, in einer besonders bevorzugten Ausführungsform bei 0,75 bis 1,5 betrieben.

Der absolute Druck in Verfahrenschritt (e) beträgt vorzugsweise 0,1 bis 10 bar, besonders bevorzugt 0,5 bis 5 bar, insbesondere 1,0 bis 2,5 bar. Die Extraktion wird vorzugsweise bei Temperaturen von -15 bis 120 °C, besonders bevorzugt 0 bis 60 °C, insbesondere 25 bis 45 °C, durchgeführt.

In einer bevorzugten Ausführungsform der vorliegenden Erfindung wird das Extraktionsmittel ausgewählt aus der Gruppe bestehend aus Cyclohexan, Methylcyclohexan, n-Hexan, n-Heptan, isomeren C6-, C7-, C8, C9-Cycloaliphaten, isomeren C6-, C7-, C8, C9-Isoaliphaten, cis-, trans-Decahydronaphthalin und Gemischen davon.

In einer besonders bevorzugten Ausführungsform wird als Extraktionsmittel Cyclohexan, Methylcyclohexan oder deren Mischungen eingesetzt. Dabei gelangt im Wesentlichen keine Lewis-Säure in die Katalysatorphase. Unter im Wesentlichen wird in diesem Zusammenhang verstanden, dass die Restkonzentration der Lewis-Säure in der Katalysatorphase vorzugsweise kleiner als 3 Gew.-%, besonders bevorzugt kleiner als 2 Gew.-%, insbesondere kleiner als 0,5 Gew.-%, bezogen auf die Katalysatorphase nach der Extraktion, ist, obwohl die besonders bevorzugten Chelatliganden mit der Lewis-Säure komplexieren und diese in die unpolare Katalysatorphase bei der Extraktion ziehen können.

Das verwendete Extraktionsmittel ist vorzugsweise wasserfrei, wobei unter wasserfrei im Sinne der vorliegenden Erfindung verstanden wird, dass das Extraktionsmittel weniger als 100 ppm, vorzugsweise weniger als 50 ppm, insbesondere weniger als 10 ppm, Wasser enthält. Das Extraktionsmittel kann durch geeignete, dem Fachmann bekannte Verfahren getrocknet werden, beispielsweise durch Adsorption oder Azeotropdestillation.

Das Extraktionsmittel wird dabei vorzugsweise in einem separaten Verfahrensschritt durch Azeotropdestillation getrocknet. Dieses erfolgt vorzugsweise destillativ als Heteroazeotropdestillation. Der absolute Druck in diesem Verfahrensschritt beträgt vorzugsweise 0,01 bis 10,0 bar, besonders bevorzugt 0,05 bis 5,0 bar, insbesondere 0,1 bis 1,0 bar. Die Destillation wird so durchgeführt, dass die Temperatur im Sumpf der Destillationsapparatur vorzugsweise 40 bis 250 °C, besonders bevorzugt 50 bis 180 °C, insbesondere 60 bis 150 °C, beträgt. Die Destillation wird so durchgeführt, dass die Temperatur am Kopf der Destillationsapparatur vorzugsweise 0 bis 200 °C, besonders bevorzugt 5 bis 100 °C, insbesondere 20 bis 50 °C, beträgt. In einer besonders bevorzugten Ausführungsform des erfindungsgemäßen Verfahrens werden die zuvor genannten Temperaturbereiche sowohl am Kopf als auch im Sumpf der Destillationsapparatur eingehalten. Die Azeotropdestillation des Extraktionsmittels erfolgt vorzugsweise in einer Destillationskolonne mit insbesondere Glockenböden, strukturierten Blechpackungen, strukturierten Gewebepackungen, Dual-Flow-Böden oder Schüttungen aus Füllkörpern als trennwirksame Einbauten, gegebenenfalls in einer Trennwandkolonne mit gegebenenfalls vorhandenen Seitenabzügen, einem Phasenabscheider am flüssigen Abzug des Kopfkondensators zur Auskreisung von Wasser, mit Apparaten für getrennte Rückführung organischer Phasen als Rücklauf auf Kolonnen, sowie weitere zur Azeotropdestillation geeigneten Apparaturen.

In dem erfindungsgemäßen Verfahren ist es darüber hinaus bevorzugt, dass der in Verfahrensschritt (e) erhaltene Nickel(0)-Katalysator, der in dem Katalysatorstrom 3 enthalten ist, zumindest teilweise in Verfahrensschritt (a) zurückgeführt wird. Hierbei ist von Vorteil, dass die Lewis-Säure in dem Verfahrensschritt (e) von dem Nickel(0)-Katalysator im Wesentlichen vollständig abgetrennt wird, da eine verbleibende Lewis-Säure in einer Hydrocyaneirung von 1,3-Butadien zu einer überproportionalen Bildung der Nebenkomponente Methylglutardinitril führt. Hierdurch würde die Gesamtselektivität des Verfahrens herabgesenkt.

Es ist von Vorteil, einen frischen bzw. reduktiv regenerierten Katalysator für das erfindungsgemäße Verfahren zunächst in der Hydrocyanierung von 3-Pentennitril einzusetzen, da in dieser Stufe eine Lewis-Säure benötigt wird und die Lewis-Säure als Nebenprodukt der reduktiven Katalysatorsynthese, zum Beispiel aus einem Gemisch von Nickelhalogenid oder Nickelhalogenid-Lösungsmitteladdukten, vorzugsweise ausgewählt aus der Gruppe bestehend aus Nickelchlorid, Nickelbromid, Nickeliodid, besonders bevorzugt Nickelchlorid und einem reduktiv wirkenden Metall, bevorzugt ausgewählt aus der Gruppe, bestehend aus Zink und Eisen, entsteht. Diesbezüglich wird auf die bereits erwähnten deutschen Patentanmeldungen DE 103 51 000.1, DE 103 51 002.8 und DE 103 51 003.6 der BASF AG verwiesen.

Besonders bevorzugt ist es daher, dass beim ersten Durchgang der Verfahrenssequenz mit Verfahrensschritt (d) unter Einsatz von frischem bzw. reduktiv regeneriertem Nickel(0)-Katalysator und frischer Lewis-Säure begonnen wird.

Es ist besonders bevorzugt, dass das in Verfahrensschritt (d) hydrocyanierte 3-Pentennitril aus dem Hydrocyanierungsstrom 1 stammt.

In einer weiteren bevorzugten Ausführungsform des erfindungsgemäßen Verfahrens wird der aus Verfahrensschritt (a) stammende Hydrocyanierungsstrom 1 vor dem Verfahrensschritt (b) zusätzlich einem Isomerisierungsschritt (a') unterzogen. Dabei wird 2-Methyl-3-butennitril, das als unerwünschtes Nebenprodukt bei der Hydrocyanierung von 1,3-Butadien entsteht und in dem Hydrocyanierungsstrom 1 enthalten ist, zu 3-Pentennitril an mindestens einem Nickel(0)-Katalysator isomerisiert. Hierbei bildet sich ein an 2-Methyl-3-butennitril abgereicherter und ein 3-Pentennitril angereicherter Isomerisierungsstrom 1. Der so erhaltene an 3-Pentennitril angereicherte Isomerisierungsstrom 1 wird anschließend anstelle des Hydrocyanierungsstroms 1 in Verfahrensschritt (b) verwendet.

Als Nickel(0)-Katalysator für die Isomerisierung von 2-Methyl-3-butennitril kann ein wie oben für die Hydrocyanierung von 1,3-Butadien oder für die Hydrocyanierung von 3-Pentennitril beschriebener Nickel(0)-Katalysator verwendet werden.

Erfindungsgemäß führt man die Isomerisierung in Gegenwart eines Systems, enthaltend
a) mindestens Nickel(0)
b) gegebenenfalls eine Nickel(0) als Ligand komplexierende, dreibindigen Phosphor enthaltende Verbindung und
c) gegebenenfalls eine Lewis-Säure
durch.

Die Herstellung von Ni(0) enthaltenden Katalysatorsystemen ist an sich bekannt und kann für die Zwecke der vorliegenden Erfindung nach an sich bekannten Verfahren erfolgen.

Weiterhin enthält das System zusätzlich eine als Ligand für Nickel(0) geeignete Verbindung, die mindestens ein dreibindiges Phosphoratom aufweist, oder ein Gemisch solcher Verbindungen. Diese Verbindung wurde in der vorliegenden Anmeldung bereits bei der Beschreibung der Hydrocyanierungskatalysatoren diskutiert.

Weiterhin enthält das System gegebenenfalls eine, wie bereits oben beschriebene, Lewis-Säure.

Erfindungsgemäß erhält man bei der Isomerisierung lineares Pentennitril.

Im Sinne der vorliegenden Verbindung werden unter dem Begriff lineares Pentennitril ein einziges solches Isomer oder eine Gemisch aus 2, 3, 4, oder 5 verschiedener solcher Isomeren verstanden.

Als lineares Pentennitril kommen cis-2-Pentennitril, trans-2-Pentennitril, cis-3-Pentennitril, trans-3-Pentennitril, 4-Pentennitril oder deren Gemische, vorzugsweise cis-3-Pentennitril, trans-3-Pentennitril, 4-Pentennitril oder deren Gemische, insbesondere cis-3-Pentennitril, trans-3-Pentennitril oder deren Gemische, die im Sinne der vorliegenden Erfindung sowohl jeweils einzeln, als auch im Gemisch als 3-Pentennitril bezeichnet werden, in Betracht.

Die Isomerisierung kann man in Gegenwart eines flüssigen Verdünnungsmittels, beispielsweise eines Kohlenwasserstoffs, wie Hexan, Heptan, Octan, Cyclohexan, Methylcyclohexan, Benzol, Decahydronaphthalin, beispielsweise eines Ethers, wie Diethylether, Tetrahydrofuran, Dioxan, Glykol-dimethylether, Anisol, beispielsweise eines Esters, wie Ethylacetat, Methylbenzoat, oder beispielsweise eines Nitrils, wie Acetonitril, Benzonitril, oder Gemischen solcher Verdünnungsmittel durchgeführt werden. In einer bevorzugten Ausführungsform kommt eine Isomerisierung in Abwesenheit eines solchen flüssigen Verdünnungsmittels in Betracht.

Weiterhin hat es sich als vorteilhaft erwiesen, alle Verfahrensschritte des erfindungsgemäßen Verfahrens in nicht-oxidierend wirkender Atmosphäre, wie unter einer Schutzgasatmosphäre aus Stickstoff oder einem Edelgas, wie Argon, durchzuführen.

Erfindungsgemäß entnimmt man gegebenenfalls während der Isomerisierung dem Reaktionsgemisch eine Mischung, enthaltend 2-Methyl-3-butennitril und lineares Pentennitril. Die Entnahme kann vorteilhaft destillativ erfolgen.

In einer bevorzugten Ausführungsform kommt die kontinuierliche oder quasikontinuierliche, vorzugsweise kontinuierliche Entnahme eines Stroms, enthaltend 2-Methyl-3-butennitril und lineares Pentennitril, vorzugsweise bestehend aus 2-Methyl-3-butennitril und lineares Pentennitril, in Betracht. Weiterhin kommt die kontinuierliche oder quasikontinuierliche, vorzugsweise kontinuierliche Zugabe des Hydrocyanierungsstroms 1, enthaltend 2-Methyl-3-butenrütril, in Betracht.

In einer bevorzugten Ausführungsform kann der zugeführte Strom einen Gehalt an 2-Methyl-3-butennitril im Bereich von 10 bis 100 Gew.-%, vorzugsweise 50 bis 75 Gew.-%, aufweisen.

In einer bevorzugten Ausführungsform kann der entnommene Strom einen Gehalt an 2-Methyl-3-butennitril von 5 bis 80 Gew.-%, vorzugsweise 20 bis 60 Gew.-%, und einen Gehalt an linearem Pentennitril von 20 bis 95 Gew.-%, vorzugsweise 40 bis 80 Gew.-%, aufweisen, mit der Maßgabe, dass die Summe der Gehalte an 2-Methyl-3-butennitril und linearem Pentennitril höchstens 100 Gew.-% beträgt.

Für die Destillation kommen hierfür übliche Apparaturen in Betracht, wie sie beispielsweise in: Kirk-Othmer, Encyclopedia of Chemical Technology, 3.Ed., Vol. 7, John Wiley & Sons, New York, 1979, Seite 870-881 beschrieben sind, wie Siebbodenkolonnen, Glockenbodenkolonnen, Packungskolonnen oder Füllkörperkolonnen.

Ebenso kommt eine Entnahme direkt aus dem Reaktor ohne Trennstufen in Betracht.

Die Zusammensetzung des entnommen Stroms hinsichtlich des molaren Verhältnisses von 2-Methyl-3-butennitril zu linearem Pentennitril kann in Abhängigkeit von der Zusammensetzung des zugeführten Stroms auf technisch einfache Weise durch die Temperatur, den Druck und das Rücklaufverhältnis bei der Destillation eingestellt werden.

In einer ersten Ausführungsform erfolgt die Isomerisierung in einer dafür konzipierten Apparateverschaltung, wie sie z.B. in der deutschen Patentanmeldung DE ... mit dem Titel "Verfahren zur Herstellung von lineare Pentennitril" der BASF AG (B03/0436) beschrieben ist.

In einer weiteren Ausführungsform erfolgt die Isomerisierung in einer oder mehreren Destillationsvorrichtungen des Verfahrensschrittes (b).

Die Temperatur im Bereich der Isomerisierungszone, die im Falle einer Destillationsvorrichtung als Reaktionsgefäß der Sumpfbereich ist, kann vorzugsweise mindestens 10°C, besonders bevorzugt mindestens 60°C, insbesondere mindestens 100°C, speziell mindestens 110°C betragen.

Die Temperatur im Bereich der Isomerisierungszone, die im Falle einer Destillationsvorrichtung als Reaktionsgefäß der Sumpfbereich ist, kann vorzugsweise höchstens 200°C, besonders bevorzugt höchstens 150°C, insbesondere höchstens 140°C, speziell höchstens 130°C betragen.

Der Druck im Bereich der Isomerisierungszone, die im Falle einer Destillationsvorrichtung als Reaktionsgefäß der Sumpfbereich ist, kann vorzugsweise mindestens 5 mbar, besonders bevorzugt mindestens 200 mbar, insbesondere mindestens 500 mbar, speziell mindestens 600 mbar betragen.

Der Druck im Bereich der Isomerisierungszone, die im Falle einer Destillationsvorrichtung als Reaktionsgefäß der Sumpfbereich ist, kann vorzugsweise höchstens 5000 mbar, besonders bevorzugt höchstens 4000 mbar, insbesondere höchstens 3000 mbar, betragen.

Ebenso kann der genannte Druck als Partialdruck des Nitrilstroms durch Einleiten eines Inertgases wie Stickstoff oder Argon eingestellt werden (Strippeffekt).

Wenn in dem erfindungsgemäßen Verfahren ein Verfahrensschritt (a') durchgeführt wird, bei dem 2-Methyl-3-butennitril isomerisiert wird, so wird in dieser Isomerisierung ein an 2-Methyl-3-butennitril abgereicherter und ein an 3-Pentennitril angereicherter Isomerisierungsstrom 1 erhalten. Aus diesem Isomerisierungsstrom 1 wird der mindestens eine Nickel(0)-Katalysator unter Erhalt eines Katalysatorstroms 1', der den mindestens einen Nickel(0)-Katalysator enthält, abgetrennt. Darüber hinaus wird ein Hydrocyanierungsstrom 2' erhalten, der 3-Pentennitril und 2-Methyl-3-butennitril enthält.

Bevorzugt erfolgt diese Trennung in dem vorhandenen Verfahrensschritt (b), so dass Katalysatorstroms 1' und Katalysatorstroms 1 sowie Hydrocyanierungsstrom 2' und Hydrocyanierungsstrom 2 identisch sind.

Dieser Hydrocyanierungsstrom 2 kann gegebenenfalls anschließend in einem Verfahrensschritt (f) aufgetrennt werden, wobei ein an 3-Pentennitril reicher Strom 4 und ein an 2-Methyl-3-butennitril reicher Strom 5 erhalten werden.

Dabei ist es bevorzugt, dass der an 3-Pentennitril reiche Strom 4, der vorzugsweise höchstens 1 Gew.-%, besonders bevorzugt höchstens 0,5 Gew.-%, insbesondere höchstens 0,3 Gew.-%, 2-Methyl-3-butennitril enthält, in Verfahrensschritt (d) geführt wird. Darüber hinaus ist bevorzugt, dass der an 2-Methyl-3-butennitril reiche Strom 5 in Verfahrensschritt (a') zurückgeführt wird, wo eine erneute Isomerisierung des 2-Methyl-3-butennitrils erfolgt.

Es ist möglich, dass nur ein Teil des Katalysatorstroms 2 für die Hydrocyanierung in Verfahrensschritt (d) verwendet wird und der restliche Teil des Katalysatorstroms 2 als By-pass-Strom 1' direkt in Verfahrensschritt (a') überführt wird. In einer weiteren Ausführungsform des erfindungsgemäßen Verfahrens werden daher die zuvor beschriebenen optionalen Verfahrensschritte (a') und (f) durchlaufen, wobei
- der Hydrocyanierungsstrom 1 direkt in Verfahrensschritt (b) geführt wird und
- der für die Isomerisierung in Verfahrensschritt (a') erforderliche Nickel(0)-Katalysator als By-pass-Strom 1' aus Verfahrensschritt (c) erhalten wird.

Alternativ ist es auch möglich, dass nur ein Teil des Katalysatorstroms 3 für die Hydrocyanierung in Verfahrensschritt (a) verwendet wird und der restliche Teil des Katalysatorstroms 3 als By-pass-Strom 2' direkt in Verfahrensschritt (a') überführt wird. In einer weiteren Ausführungsform des erfindungsgemäßen Verfahrens werden daher die zuvor beschriebenen optionalen Verfahrensschritte (a') und (f) durchlaufen, wobei
- der Hydrocyanierungsstrom 1 direkt in Verfahrensschritt (b) geführt wird und
- der für die Isomerisierung in Verfahrensschritt (a') erforderliche Nickel(0)-Katalysator als By-pass-Strom 2 aus Verfahrensschritt (e) erhalten wird.

Darüber hinaus ist es bevorzugt, dass in dem erfindungsgemäßen Verfahren in Verfahrensschritt (c) ein frischer bzw. reduktiv regenerierter Nickel(0)-Katalysator zugeführt wird.

Besonders bevorzugt ist, wenn das erfindungsgemäße Verfahren kontinuierlich durchgeführt wird.

Durch die zuvor beschriebene Schließung des Katalysatorkreislaufes beträgt der Nickel(0)-Verlust vor der Regenerierung vorzugsweise zwischen 30 und 80 %, besonders bevorzugt 30 bis 70 %, insbesondere 40 bis 60 %, des ursprünglichen Wertes. Damit ist gewährleistet, dass die Katalysatorregeneration stets genug Lewis-Säure für die Hydrocyanierung von 3-Pentennitril bildet, vorzugsweise 0,4 bis 8 Mol-Äquivalente/Nickel, besonders bevorzugt 0,4 bis 4 Mol-Äquivalente/Nickel, insbesondere 0,4 bis 1 Mol-Äquivalente/Nickel.

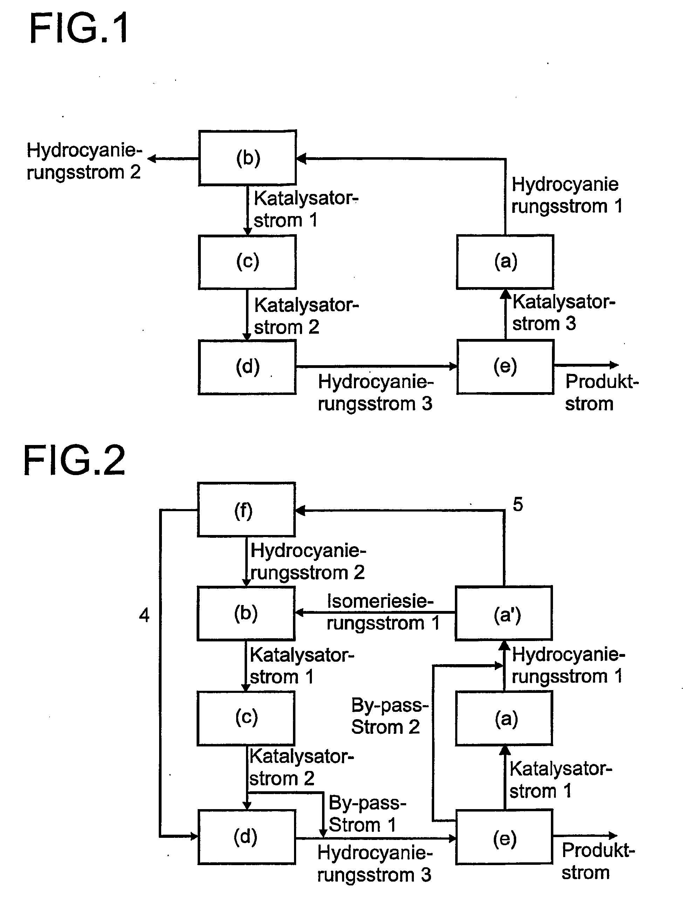

Die vorliegende Erfindung wird anhand der Figuren 1 bis 4 näher erläutert.

Figur 1 zeigt eine bevorzugte Ausführungsform des erfindungsgemäßen Verfahrens, wobei der aus Verfahrensschritt (a) stammende Hydrocyanierungsstrom I keiner Isomerisierung unterzogen wird.

In Verfahrensschritt (a) findet zunächst eine Hydrocyanierung von 1,3-Butadien mit Cyanwasserstoff statt. Aus diesem Verfahrensschritt (a) resultiert ein Hydrocyanierungsstrom 1, der in den Verfahrensschritt (b) der Katalysatorabtrennung geführt wird. In diesem Verfahrensschritt (b) erfolgt eine Abtrennung des mindestens einen Nickel(0)-Katalysators aus dem Hydrocyanierungsstrom 1 unter Erhalt eines Katalysatorstromes 1. Dieser Katalysatorstrom 1 enthält den mindestens einen Nickel(0)-Katalysator. Darüber hinaus wird ein Hydrocyanierungsstrom 2 in Verfahrensschritt (b) erhalten, der 3-Pentennitril, 2-Pentennitril, 2-Methyl-2-butennitril, 2-Methyl-3-butennitril und C₉-Nitrile enthält. Der Katalysatorstrom 2 wird anschließend in den Verfahrensschritt (c) überführt. Hier erfolgt eine reduktive Regenerierung des mindestens einen Nickel(0)-Katalysators in dem Katalysatorstrom 1. Es resultiert ein Katalysatorstrom 2, der anschließend in den Verfahrensschritt der Hydrocyanierung von 3-Pentennitril überführt wird (Verfahrensschritt (d)). Aus dieser Hydrocyanierung wird ein Hydrocyanierungsstrom 3 erhalten, der in die Katalysatorabtrennung des Verfahrensschrittes (e) überführt wird. Hierbei wird ein Katalysatorstrom 3 erhalten, der in den Verfahrensschritt (a) der Hydrocyanierung von 1,3-Butadien zurückgeführt wird. Darüber hinaus wird in Verfahrensschritt (e) ein Produktstrom erhalten, der Adipodinitril enthält.

Figur 2 verdeutlicht eine Ausführungsform des erfindungsgemäßen Verfahrens, bei dem eine Isomerisierung von 2-Methyl-3-butennitril zu 3-Pentennitril erfolgt.

Im Verfahrensschritt (a) erfolgt eine Hydrocyanierung von 1,3-Butadien an mindestens einen Nickel(0)-Katalysator. Hieraus resultiert ein Hydrocyanierungsstrom 1, der 3-Pentennitril, 2-Pentennitril, 2-Methyl-2-butennitril, 2-Methyl-3-butennitril, C₉-Nitrile, Methylglutardinitril und den mindestens einen Nickel(0)-Katalysator enthält. Dieser Hydrocyanierungsstrom wird anschließend in einen Verfahrensschritt (a') überführt, in dem eine Isomerisierung von 2-Methyl-3-butennitril zu 3-Pentennitril an mindestens einem Nickel(0)-Katalysator erfolgt. Hierbei wird ein an 2-Methyl-3-butennitril abgereicherter und ein an 3-Pentennitril angereichter Isomerisierungsstrom 1 erhalten. Dieser Isomerisierungsstrom 1 wird anschließend in den Verfahrensschritt (b) überführt, In diesem Verfahrensschritt (b) erfolgt eine Abtrennung des mindestens einen Nickel(0)-Katalysators aus dem Isomerisierungsstrom 1 unter Erhalt eines Katalysatorstromes 1, der den mindestens einen Nickel(0)-Katalysator enthält. Darüber hinaus wird in diesem Verfahrensschritt ein Hydrocyanierungsstrom 2 erhalten, der 3-Pentennitril, 2-Pentennitril, 2-Methyl-2-butennitril, C₉-Nitrile und 2-Methyl-3-butennitril enthält. Anschließend wird der Katalysatorstrom 1 in den Verfahrensschritt (c) überführt und reduktiv regeneriert. Hierbei wird ein Katalysatorstrom 2 erhalten, der teilweise in den Verfahrensschritt (d) überführt wird. Der nicht in Verfahrensschritt (d) überführte Katalysatorstrom wird als By-pass-Strom 1 dem aus Verfahrensschritt (d) resultierenden Hydrocyanierungsstrom 3 zugefügt. In Verfahrensschritt (d) erfolgt eine Hydrocyanierung von 3-Pentennitril an dem mindestens einen Nickel(0)-Katalysator in Gegenwart einer Lewis-Säure, der in dem Katalysatorstrom 2 enthalten ist. Aus dem Verfahrensschritt (d) resultiert ein Hydrocyanierungsstrom 3, der Adipodinitril, den mindestens einen Nickel(0)-Katalysator und die mindestens eine Lewis-Säure enthält. Dieser Hydrocyanierungsstrom 3 wird anschließend in den Verfahrensschritt (e) zur Abtrennung des mindestens einen Nickel(0)-Katalysators überführt. Diese Abtrennung erfolgt durch Extraktion mit einem organischen Lösemittel. Es wird ein Katalysatorstrom 3 erhalten, der zum Teil in den Verfahrensschritt (a) zurückgeführt wird. Der nicht zurückgeführte Teil des Katalysatorstromes 3 wird als By-pass-Strom 2 direkt in den Hydrocyanierungsstrom 1 geleitet. Im Verfahrensschritt (e) wird schließlich noch ein Produktstrom erhalten, der Adipodinitril enthält. Zusätzlich wird der in Verfahrensschritt (b) erhaltene Hydrocyanierungsstrom 2 in einem Verfahrensschritt (f) in einen an 2-Methyl-3-butennitril reichen Strom 5 und einen an 3-Pentennitril reichen Strom 4 aufgetrennt. Der Strom 4 wird in den Verfahrensschritt (d) geführt und der Strom 5 in den Verfahrensschritt (a').

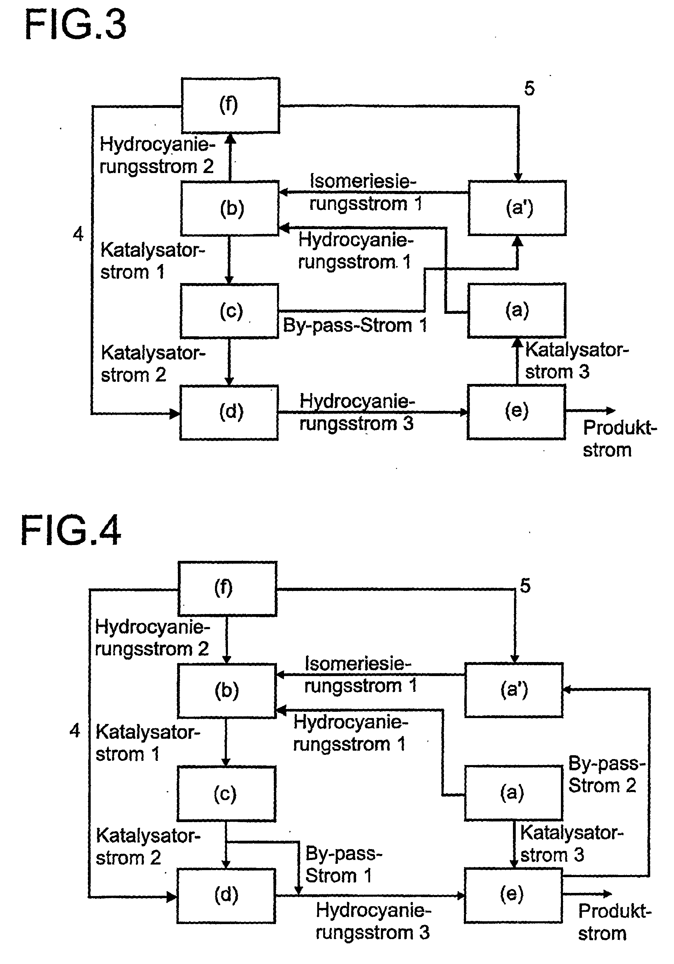

Figur 3 verdeutlicht eine Ausführungsform des erfindungsgemäßen Verfahrens, bei dem eine Isomerisierung von 2-Methyl-3-butennitril zu 3-Pentennitril erfolgt.

Im Verfahrensschritt (a) erfolgt eine Hydrocyanierung von 1,3-Butadien an mindestens einem Nickel(0)-Katalysator. Hieraus resultiert ein Hydrocyanierungsstrom 1, der 3-Pentennitril, 2-Pentennitril, 2-Methyl-2-butennitril, 2-Methyl-3-butennitril, C₉-Nitrile, Methylglutardinitril, den mindestens einen Nickel(0)-Katalysator, nicht umgesetztes 1,3-Butadien und Reste nicht umgesetzten Cyanwasserstoffs enthält. Dieser Hydrocyanierungsstrom wird anschließend in einen Verfahrensschritt (b) überführt. In diesem Verfahrensschritt (b) erfolgt eine Abtrennung des mindestens einen Nickel(0)-Katalysators aus dem Hydrocyanierungsstrom 1 unter Erhalt eines Katalysatorstroms 1, der den mindestens einen Nickel(0)-Katalysator enthält. Darüber hinaus wird in diesem Verfahrensschritt ein Hydrocyanierungsstrom 2 erhalten, der 3-Pentennitril, 2-Pentennitril, 2-Methyl-2-butennitril, C₉-Nitrile und 2-Methyl-3-butennitril enthält. Dieser erhaltene Hydrocyanierungsstrom 2 wird anschließend in einen Verfahrensschritt (f) in einen an 2-Methyl-3-butennitril reichen Strom 5 und einen an 3-Pentennitril reichen Strom 4 aufgetrennt. Der Strom 4 wird in den Verfahrensschritt (d) geführt und der Strom 5 wird in einen Isomerisierungsschritt (a') geführt. In diesem Verfahrensschritt (a') findet eine Isomerisierung von 2-Methyl-3-butennitril zu 3-Pentennitril an mindestens einem Nickel(0)-Katalysator statt. Hierbei wird ein an 2-Methyl-3-butennitril abgereicherter und ein an 3-Pentennitril angereicherter Isomerisierungsstrom 1 erhalten. Dieser Isomerisierungsstrom 1 wird anschließend in den Verfahrensschritt (b) überführt. Der für die Isomerisierung erforderliche Nickel(0)-Katalysator in dem Verfahrensschritt (a') wird aus dem weiter unten beschriebenen Verfahrensschritt (c) als By-pass-Strom 1' erhalten. Der in Verfahrensschritt (b) erhaltene Katalysatorstrom wird in den Verfahrensschritt (c) überführt und reduktiv regeneriert. Hierbei wird ein Katalysatorstrom 2 erhalten, der teilweise in den Verfahrensschritt (d) überführt wird. Der nicht in Verfahrensschritt (d) überführte Teil des Katalysatorstrom 2 wird als By-pass-Strom 1', so wie zuvor beschrieben, dem Verfahrensschritt (a') zugefügt. In Verfahrensschritt (d) erfolgt eine Hydrocyanierung von 3-Pentennitril an dem mindestens einen Nickel(0)-Katalysator in Gegenwart einer Lewis-Säure, die in dem Katalysatorstrom 2 enthalten ist. Aus dem Verfahrensschritt (d) resultiert ein Hydrocyanierungsstrom 3, der Adipodinitril, den mindestens einen Nickel(0)-Katalysator und die mindestens eine Lewis-Säure enthält. Dieser Hydrocyanierungsstrom 3 wird anschließend in den Verfahrensschritt (e) zur Abtrennung des mindestens einen Nickel(0)-Katalysators überführt. Diese Abtrennung erfolgt durch Extraktion mit einem organischen Lösemittel. Es wird ein Katalysatorstrom 3 erhalten, der in den Verfahrensschritt (a) zurückgeführt wird. Figur 4 verdeutlicht eine Ausführungsform des erfindungsgemäßen Verfahrens, bei dem eine Isomerisierung von 2-Methyl-3-butennitril zu 3-Pentennitril erfolgt.

Im Verfahrensschritt (a) erfolgt eine Hydrocyanierung von 1,3-Butadien an mindestens einem Nickel(0)-Katalysator. Hieraus resultiert ein Hydrocyanierungsstrom 1, der 3-Pentennitril, 2-Pentennitril, 2-Methyl-2-butennitril, 2-Methyl-3-butennitril, C₉-Nitrile, Methylglutardinitril, den mindestens einen Nickel(0)-Katalysator, nicht umgesetztes 1,3-Butadien und Reste nicht umgesetzten Cyanwasserstoffs enthält. Dieser Hydrocyanierungsstrom wird anschließend in einen Verfahrensschritt (b) überführt. In diesem Verfahrensschritt (b) erfolgt eine Abtrennung des mindestens einen Nickel(0)-Katalysators aus dem Hydrocyanierungsstrom 1 unter Erhalt eines Katalysatorstroms 1, der den mindestens einen Nickel(0)-Katalysator enthält. Darüber hinaus wird in diesem Verfahrensschritt ein Hydrocyanierungsstrom 2 erhalten, der 3-Pentennitril, 2-Pentennitril, 2-Methyl-2-butennitril, C₉-Nitrile und 2-Methyl-3-butennitril enthält. Dieser erhaltene Hydrocyanierungsstrom 2 wird anschließend in einen Verfahrensschritt (f) in einen an 2-Methyl-3-butennitril reichen Strom 5 und einen an 3-Pentennitril reichen Strom 4 aufgetrennt. Der Strom 4 wird in den Verfahrensschritt (d) geführt und der Strom 5 wird in einen Isomerisierungsschritt (a') geführt. In diesem Verfahrensschritt (a') findet eine Isomerisierung von 2-Methyl-3-butennitril zu 3-Pentennitril an mindestens einem Nickel(0)-Katalysator statt. Hierbei wird ein an 2-Methyl-3-butennitril abgereicherter und ein an 3-Pentennitril angereicherter Isomerisierungsstrom 1 erhalten. Dieser Isomerisierungsstrom 1 wird anschließend in den Verfahrensschritt (b) überführt. Der für die Isomerisierung erforderliche Nickel(0)-Katalysator in dem Verfahrensschritt (a') wird aus dem weiter unten beschriebenen Verfahrensschritt (e) als By-pass-Strom 2 erhalten. Der in Verfahrensschritt (b) erhaltene Katalysatorstrom wird in den Verfahrensschritt (c) überführt und reduktiv regeneriert. Hierbei wird ein Katalysatorstrom 2 erhalten, der teilweise in den Verfahrensschritt (d) überführt wird. Der nicht in Verfahrensschritt (d) überführte Katalysatorstrom wird als By-pass-Strom 1 dem aus Verfahrensschritt (d) resultierenden Hydrocyanierungsstrom 3 zugefügt. In Verfahrensschritt (d) erfolgt eine Hydrocyanierung von 3-Pentennitril an dem mindestens einen Nickel(0)-Katalysator in Gegenwart einer Lewis-Säure, die in dem Katalysatorstrom 2 enthalten ist. Aus dem Verfahrensschritt (d) resultiert ein Hydrocyanierungsstrom 3, der Adipodinitril, den mindestens einen Nickel(0)-Katalysator und die mindestens eine Lewis-Säure enthält. Dieser Hydrocyanierungsstrom 3 wird anschließend in den Verfahrensschritt (e) zur Abtrennung des mindestens einen Nickel(0)-Katalysators überführt. Diese Abtrennung erfolgt durch Extraktion mit einem organischen Lösemittel. Es wird ein Katalysatorstrom 3 erhalten, der zum Teil in den Verfahrensschritt (a) zurückgeführt wird. Der nicht zurückgeführte Teil des Katalysatorstroms 3 wird als By-pass-Strom 2, sowie bereits oben erwähnt, direkt in den Verfahrensschritt (a') geleitet.

### Beispiele

### Abkürzungen:

| | |
|---|---|
| 3PN | lineare Pentennitril-Isomere (trans-3-, cis-3-, trans-2-, cis-2-, 4-Pentennitril) |
| C5-Abfallnitrile | Valeriansäurenitril, E- und Z-2-Methyl-2-butennitril |
| ADN | Adipiodinitril |
| MGN | Summe aus 2-Methylglutar und 2-Ethylbernsteinsäuredinitril |
| 2M3BN | 2-Methyl-3-butennitril |
| P | Phosphor |
| Ni(0) | Nickel der Oxidationsstufe 0 |
| BD | 1,3-Butadien |
| BU | Summe aus 1-Buten, cis- und trans-2-Buten |
| C2BU | cis-2-Buten |
| HCN | Cyanwasserstoff |
| LL | Chelatligand der Formel 1 |
| NiLL | Ni(0)-Komplex des Chelatliganden LL |
| TBC | tert-Butylbrenzkatechin |

Alle Angaben bzgl. Konzentrationen sind in Gew.-% aufgeführt.

Die Konzentrationen an C5- und C6-Nitrilen, BD und BU werden per GC mit internem Standard (Benzonitril) bestimmt, HCN durch Absorption in NaOH und anschließende Cyanid-Titration quantifiziert. Ni(0) wird durch Cyclovoltametrie bestimmt, P durch A-tomabsorptionsspektroskopie.

### Beispiel 1:

In Beispiel 1 wird für beide Hydrocyanierungen von Butadien zu Adipodinitril ein einziges Katalysatorsystem auf Basis von Nickel(0)-Komplexen mit Chelatligand 1 eingesetzt.

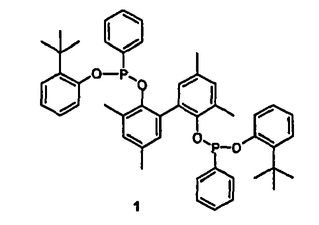

Der Ligand 1 (LL) wird gegenüber Ni(0) im Überschuss eingesetzt, so dass NiLL und freier LL vorliegen. In der zweiten Hydrocyanierung wird zusätzlich ZnCl₂ benötigt. Der Katalysatorkreislauf über sieben Verfahrensschritte wird nachfolgend beschrieben.

In einem Schritt (1) werden folgende Ströme in einen Schlaufenreaktor R1 von 25 l Volumen gefahren, der mit einer Düse, Impulsaustauschrohr, externen Umpumpkreislauf und einem im Umpumpkreislauf befindlichen Wärmetauscher zur Abfuhr der Reaktionsenergie ausgestattet ist und auf 367 K temperiert ist:
a) 5,7 kg/h flüssiger unstabilisierter, durch Destillation von Wasser befreiter Cyanwasserstoff (Strom 1),
b) 15,8 kg/h Gemisch aus BD und BU mit ca. 90% BD-Gehalt (Strom 6), das erhalten wird aus dem rückgeführten Strom 5, dessen Gewinnung in Schritt (2) beschrieben wird und beigemischten 11,5 kg/h handelsüblichem BD (Strom 2), enthaltend 0,25 % C2BU, das durch Kontakt mit Aluminiumoxid behandelt wurde, um Wasser und Stabilisator TBC zu entfernen,
c) 5,4 kg/h Nickel(0)-Katalysatorlösung, erhalten wie in diesem Beispiel in Schritt (2) beschrieben als Teilstrom 3 im Sumpf der K2.

Der aus dem Reaktor R1 abgezogene Strom 4 (27 kg/h) enthält in Summe 16,2 % BD und BU, entsprechend einem Umsatz von 80,4 % BD, sowie 35,0 % 3PN, 29,8 % 2M3BN und geringe Mengen C5-Abfall-Nitrile. Zudem ergibt die Ni(0)-Analyse 0,4 % und die P-Analyse 1,2 %. ADN ist zu 1,0 % enthalten, zudem 1,0 % MGN.

Strom 4 wird in einem Schritt (2) einer Destillationskolonne K1 zugeführt, die mit Verstärkungs- und Abtriebsteil betrieben wird und mit einem Fallfilmverdampfer und getrenntem Sumpf ausgestattet ist, sowie Kolonneneinbauten mit strukturierter Packung enthält, die 10 theoretische Trennstufen erzeugen. Kolonne K1 wird am Kopf mit einem Direktkondensator betrieben der aus einem mit strukturierter Packung bestückten Kolonnenschuss mit Totalfangtasse, Umpumpkreis und externem Wärmetauscher besteht. Die Kolonne K1 wird bei einem absoluten Kopfdruck von 2,0 bar, 288 K Kopftemperatur und 363 K Sumpfabzugstemperatur betrieben.

Über Kopf der Kolonne K1 wird der Strom 5 erhalten, der zusammen mit dem Frisch-BD-Strom 2 wie zuvor beschrieben als Rückführstrom 6 in den Reaktor R1 dosiert wird. Das Rücklaufverhältnis am Kopf der Kolonne K1 wird so eingestellt, dass der Strom 5 ca. 100 ppm 2M3BN enthält.

Über Sumpf der Kolonne K1 werden 24,9 kg/h eines Stromes 7 erhalten, der 3,1 % BD, 5,4 % BU, 38,0 % 3PN und 32,8 % 2M3BN sowie zusätzlich die Katalysatorbestandteile enthält (Analyse: 0,4 % Ni(0), 1,3 % P). BU ist im Verhältnis zu BD gegenüber dem Zulauf deutlich angereichert.

Im Sumpf der Kolonne K1 wird zudem ein Strom 9 (2,2 kg/h) eingespeist, der in der nachfolgenden Kolonne K2 erzeugt wird.

Strom 7 wird innerhalb des Schrittes (2) in eine Destillationskolonne K2 gefahren, die in Abtriebsfahrweise betrieben wird und mit Fallfilmverdampfer, Kopfkondensator mit Nachkondensator sowie Kolonneneinbauten mit strukturierter Packung ausgestattet ist, die 10 theoretische Trennstufen erzeugen. Die Kolonne wird bei einem absoluten Kopfdruck von 150 mbar, 329 K Kopftemperatur und 373 K Sumpfabzugstemperatur betrieben. Der Brüdenstrom der Kolonne wird bei 308 K teilkondensiert und mit einem Nachkondensator bei 263 K behandelt. Der so von 2M3BN und anderen Pentennitrilen abgereicherte BD-Strom 8 (2,3 kg/h) wird in einem Verdichter V1 auf einen absoluten Druck von 1,2 bar verdichtet (34,3 % BD, 59,1 % BU, Rest C5-Nitrile). Der verdichtete Gasstrom wird bei 279 K zum großen Teil unter Erhalt eines Stromes 9 (s. oben) kondensiert, wobei der Gasstrom 10 (41,5 Nl/h, enthaltend 56,2 % BU) anfällt und entsorgt wird. Strom 9 wird flüssig in den Sumpf der Kolonne K1 zurückgefahren.

An der Kolonne K2 wird in einem gasförmigen Seitenabzug der Strom 11 gewonnen (24,9 kg/h), enthaltend ca. 100 ppm BD, 32,2 % 2M3BN und 63,4 % 3PN sowie 3,4 % C5-Abfall-Nitrile. Die Position des Seitenabzugs ist so gewählt, dass unter dem Seitenabzug in einem Abtriebsteil die Komponente 2M3BN in dem über Sumpf gewonnen Strom 13 im Verhältnis zu 3PN abgereichert wird (ca. 4 theoretische Stufen unterhalb des Kopfes). Die Aufarbeitung dieses Stromes wird z.B. in DE-A-102 004 004 671 Bsp. 1 für den dortigen Strom 11 beschrieben.

In den Sumpf der Kolonne K2 werden 21,9 kg/h des Katalysatorstromes 12 gefahren, dessen Herstellung weiter unten in Schritt (7) beschrieben wird.

An der Kolonne K2 wird über Sumpf der Katalysator-Strom 13 erhalten, enthaltend 2,1 % Ni(0), 6,0 % P, ca. 100 ppm 2M3BN und 16,7 % 3PN, zudem ca. 1,0 % Abfall-C5-Nitrile und 6,8 % C6-Dinitrile, wovon 25 % MGN darstellen. Der Strom 13 wird aufgespalten in Teilstrom 3 (5,4 kg/h), der wie zuvor beschrieben in den Reaktor R1 zurückgefahren wird. Der andere Teil (Strom 14) (14,1 kg/h) wird einer Regenerierung in Schritt (3) zugeführt.

In Schritt (3) wird in einem Rührbehälter R3 (50 L, Doppelmantel-Glasbehälter) durch Zufuhr von 15,7 kg/h NiCl₂-Suspension (Strom 17; 3,5 Gew.-% in Pentennitril; umgepumpt; entnommen aus der Umpumpleitung des Vorratsgefäßes; hergestellt z.B. wie in Beispiel 6 der Anmeldung DE-A-103 51 002 beschrieben) und Zn-Pulver in fester Form (mittlerer Partikeldurchmesser 11 µm, Dosierung über eine Teflonschnecke aus einem Silo; Strom 16; 0,3 kg/h) in einer Redox-Reaktion zusätzliches Ni(0) erzeugt. In situ wird dieses Ni(0) mit LL aus Strom 15 (0,2 kg/h) komplexiert. In der Umsetzung entsteht zudem stöchiometrisch eine entsprechende Menge ZnCl₂. Der Reaktor wird auf 80 °C temperiert. Der Behälter ist mit Stickstoff überdeckt.

Der aus Schritt (3) austretende Strom 18 wird in einem Schritt (4) einem Schlaufenreaktor R3 von 250 L Volumen, der mit einer Strahldüse, Impulsaustauschrohr, externem Umpumpkreislauf und Wärmetauscher zur Abfuhr der Reaktionswärme ausgestattet ist, zugeführt. Zudem werden folgende Ströme dosiert:
a) 10,0 kg/h flüssiger, unstabilisierter, durch Destillation von Wasser befreiter Cyanwasserstoff (Strom 20),
b) 56,5 kg/h Pentennitril (Strom 19), enthaltend 83,3% 3PN und Rest C5-Abfall-Nitrile

Der aus dem Reaktor R3 abgezogene Strom 21 (96,7 kg/h) enthält 36,0 % 3PN sowie 39,4 % ADN und 2,5 % MGN, entsprechend einem Umsatz von 54% Pentennitril.

Strom 23 wird in einem Schritt (5) einer einstufigen Destillation in einem umgepumpten Behälter B2 mit außenliegendem Zwangsumlaufentspannungsverdampfer und einem Kopfkondensator zugeführt. Der Behälter B2 wird bei 25 mbar absolutem Kopfdruck, 313 K Kondensationstemperatur und 343 K Sumpfabzugstemperatur betrieben.

Über Kopf des Behälters B2 wird der Strom 22 erhalten (32,5 kg/h), der in Summe 77,4 % 3PN sowie 21,9 % Abfall-C5-Nitrile enthält, der Rest sind C6-Dinitrile. Strom 25 wird im folgenden aufgearbeitet wie z.B. in DE-A-102 004 004 683, Bsp. 2 für den dortigen Strom 4 beschrieben.

Über Sumpf des Behälters B2 werden 64,4 kg/h eines Stromes 23 mit einem Gehalt von 15,1 % 3PN sowie 3,9 % C5-Abfallnitrile erhalten. Der C6-Dinitril-Gehalt beträgt 59,0 % bzgl. ADN und 3,6 % bzgl. MGN. Strom 23 enthält zudem den Katalysatorkomplex und freien Liganden (Ni(0) 0,5 %; P 1,3 %), ZnCl₂ (Cl-Analyse 0,5 %) sowie in geringem Maß Katalysatorabbauprodukte.

Strom 23 wird in einem Schritt (6) am oberen Ende einer Gegenstromextraktionskolonne K3 aufgegeben und mit 96,2 kg/h eines Stromes 24, enthaltend 92,8 % Methylcyc-Iohexan als Extraktionsmittel sowie verschiedene Pentennitril-Isomere, extrahiert, dessen Herstellung weiter unten beschrieben wird. Der am Kopf der Extraktion erhaltene Strom 26 besteht zu 83,3 % aus dem Extraktionsmittel und enthält neben 4,0 % 3PN, 0,7 % ADN und die Katalysatorkomponenten Nickel(0)-Komplex und freien Liganden, so dass 0,3 % Ni(0) und 0,9 % P gemessen werden. Das Zinkchlorid verbleibt vollständig im Sumpfabzug der Extraktionskolonne in Strom 25. Strom 25 wird im folgenden aufgearbeitet wie z.B. in DE-A-102 004 004 683, Bsp. 2 für den dortigen Strom 7 beschrieben, um unumgesetztes Pentennitril und enthaltenes MCH zurückzugewinnen und das Reinprodukt ADN zu isolieren.

Strom 26 wird in einem Schritt (7) in eine Destillationskolonne K4 gefahren, die mit Fallfilmverdampfer, einem geteilten Sumpf und Kopfkondensator sowie Kolonneneinbauten mit strukturierter Packung ausgestattet ist, die 12 theoretische Trennstufen erzeugen. Die Kolonne wird bei 100 mbar absolutem Kopfdruck, 308 K Kopftemperatur und 353 K Sumpfabzugstemperatur betrieben. Im Sumpf wird Pentennitril (11,7 kg/h; Strom 30) zugefahren, das genauso hergestellt wurde wie das Pentennitril in Strom 19. Zudem wird das MCH aus der Aufarbeitung von Strom 25 zurückgeführt (9,6 kg/h, 88,8% MCH, 9,5 % 3PN, Rest Abfall-C5-Nitrile) und als Strom 29 in die Kolonne K4 eingespeist.

Über Kopf der Kolonne K4 wird das Extraktionsmittel zurückgewonnen, das zusammen mit einem kleinen makeup-Strom 28 von reinem, auf eine Wasserkonzentration unter 10 ppm getrocknetem MCH als Strom 26 in die Extraktionskolonne K3 zurückgefahren wird.

Am Sumpf der Kolonne K4 wird in Strom 12 der extrahierte Katalysator als Lösung in Pentennitril gewonnen (21,9 kg/h), dessen Einspeisung in K2 in Schritt (2) bereits beschrieben wurde. Dieser Strom enthält 43,9 % 3PN, 3,5 % C5-Abfall-Nitrile, 3,3 % ADN und 0,3 % MGN. Der Ni(0)-Gehalt wird mit 1,4 % und der P-Gehalt mit 3,9 % bestimmt. Der MCH-Gehalt in Strom 12 wird in der Kolonne K4 auf 10 Gew.-ppm eingestellt.

## Patentansprüche

1. Verfahren zur Herstellung von Adipodinitril durch Hydrocyanierung von 1,3-Butadien an einem Katalysator, wobei
- in einem ersten Verfahrensschritt 1,3-Butadien zu 3-Pentennitril an mindestens einem Nickel(0)-Katalysator hydrocyaniert wird und
- in einem zweiten Verfahrensschritt 3-Pentennitril zu Adipodinitril an mindestens einem Nickel(0)-Katalysator unter Zugabe mindestens einer Lewis-Säure hydrocyaniert wird,
**dadurch gekennzeichnet,**
**dass** zumindest einer der in den jeweiligen Verfahrensschritten
(a) Hydrocyanierung von 1,3-Butadien an mindestens einem Nickel(0)-Katalysator, wobei ein Hydrocyanierungsstrom 1 resultiert, der 3-Pentennitril, 2-Pentennitril, 2-Methyl-2-butennitril, C₉-Nitrile, 2-Methyl-3-butennitril, Methylglutardinitril, den mindestens einen Nickel(0)-Katalysator, nicht umgesetztes 1,3-Butadien und Reste nicht umgesetzten Cyanwasserstoffs enthält,
(b) Abtrennung des mindestens einen Nickel(0)-Katalysators aus dem Hydrocyanierungsstrom 1 unter Erhalt eines Katalysatorstromes 1, der den mindestens einen Nickel(0)-Katalysator enthält, und eines Hydrocyanierungsstroms 2, der 3-Pentennitril, 2-Pentennitril, 2-Methyl-2-butennitril, C₉-Nitrile und 2-Methyl-3-butennitril enthält,
(c) Regeneration des mindestens einen Nickel(0)-Katalysators in dem Katalysatorstrom 1 durch reduktive Nickel-Katalysatorsynthese unter Zugabe von frischem Ligand unter Erhalt eines Katalysatorstroms 2,
(d) Hydrocyanierung von 3-Pentennitril an mindestens einem Nickel(0)-Katalysator und in Gegenwart mindestens einer Lewis-Säure, wobei der Nickel(0)-Katalysator und die Lewis-Säure zumindest teilweise aus dem Katalysatorstrom 2 stammen und ein Hydrocyanierungsstrom 3 resultiert, der den mindestens einen Nickel(0)-Katalysator, Adipodinitril und die mindestens eine Lewis-Säure enthält,
(e) Abtrennung des mindestens einen Nickel(0)-Katalysators aus dem Hydrocyanierungsstrom 3 durch Extraktion mit einem organischen Lösemittel unter Erhalt eines Katalysatorstroms 3, der den mindestens einen Nickel(0)-Katalysator enthält, und eines Produktstromes, der Adipodinitril enthält, wobei der Katalysatorstrom 3 zumindest teilweise in Verfahrensschritt (a) zurückgeführt werden kann,
verwendeten Nickel(0)-Katalysatoren zumindest teilweise in den jeweils anderen Verfahrensschritt überführt wird.

2. Verfahren nach Anspruch 1, **gekennzeichnet durch** die folgenden Verfahrensschritte:
(a) Hydrocyanierung von 1,3-Butadien an mindestens einem Nickel(0)-Katalysator, wobei ein Hydrocyanierungsstrom 1 resultiert, der 3-Pentennitril, 2-Pentennitril, 2-Methyl-2-butennitril, C₉-Nitrile, Methylglutarnitril, 2-Methyl-3-butennitril, den mindestens einen Nickel(0)-Katalysator, nicht umgesetztes 1,3-Butadien und Reste nicht umgesetzten Cyanwasserstoffs enthält,
(a') Isomerisierung von 2-Methyl-3-butennitril, das in dem Hydrocyanierungsstrom 1 enthalten ist, zu 3-Pentennitril an mindestens einem Nickel(0)-Katalysator, wobei ein an 2-Methyl-3-butennitril abgereicherter und ein an 3-Pentennitril angereicherter Isomerisierungsstrom 1 erhalten wird,
(b) Abtrennung des mindestens einen Nickel(0)-Katalysators aus dem Isomerisierungsstrom 1 unter Erhalt eines Katalysatorstromes 1, der den mindestens einen Nickel(0)-Katalysator enthält, und eines Hydrocyanierungsstroms 2, der 3-Pentennitril, 2-Pentennitril, 2-Methyl-2-butennitril, C₉-Nitrile und 2-Methyl-3-butennitril enthält,
(c) Regeneration des mindestens einen Nickel(0)-Katalysators in dem Katalysatorstrom 1 **durch** reduktive Nickel-Katalysatorsynthese unter Zusatz von frischem Ligand unter Erhalt eines Katalysatorstroms 2,
(d) Hydrocyanierung von 3-Pentennitril an mindestens einem Nickel(0)-Katalysator und in Gegenwart mindestens einer Lewis-Säure, wobei der Nickel(0)-Katalysator und die Lewis-Säure zumindest teilweise aus dem Katalysatorstrom 2 stammen und ein Hydrocyanierungsstrom 3 resultiert, der Adipodinitril, den mindestens einen Nickel(0)-Katalysator und die mindestens eine Lewis-Säure enthält,
(e) Abtrennung des mindestens einen Nickel(0)-Katalysators aus dem Hydrocyanierungsstrom 2 **durch** Extraktion mit einem organischen Lösemittel unter Erhalt eines Katalysatorstroms 3, der den mindestens einen Nickel(0)-Katalysator enthält, und eines Produktstromes, der Adipodinitril enthält, wobei der Katalysatorstrom 3 zumindest teilweise in Verfahrensschritt (a) zurückgeführt werden kann.

3. Verfahren nach Anspruch 2, **dadurch gekennzeichnet, dass** in einem zusätzlichen Verfahrensschritt (f) der Hydrocyanierungsstrom 2 aufgetrennt wird und ein an 3-Pentennitril reicher Strom 4 und ein an 2-Methyl-3-butennitril reicher Strom 5 erhalten werden.

4. Verfahren nach Anspruch 3, **dadurch gekennzeichnet, dass** der an 3-Pentennitril reiche Strom 4 in Verfahrensschritt (d) geführt wird.

5. Verfahren nach Anspruch 3 oder 4, **dadurch gekennzeichnet, dass** der an 2-Methyl-3-butennitril reiche Strom 5 in Verfahrensschritt (a') geführt wird.

6. Verfahren nach einem der Ansprüche 1 bis 5, **dadurch gekennzeichnet, dass** beim ersten Durchgang der Verfahrenssequenz mit Verfahrensschritt (d) unter Einsatz von frischer Lewis-Säure und frischem Nickel(0)-Katalysator und/oder reduktiv regeneriertem Nickel(0)-Katalysator begonnen wird.

7. Verfahren nach einem der Ansprüche 1 bis 6, **dadurch gekennzeichnet, dass** das in Verfahrensschritt (d) hydrocyanierte 3-Pentennitril aus dem Hydrocyanierungsstrom 1 oder dem Isomerisierungsstrom 1 stammt.

8. Verfahren nach einem der Ansprüche 1 bis 7, **dadurch gekennzeichnet, dass** nur ein Teil des Katalysatorstroms 2 für die Hydrocyanierung in Verfahrensschritt (d) verwendet wird und der restliche Teil des Katalysatorstroms 2 als By-pass-Strom 1 direkt in Verfahrensschritt (e) überführt wird.

9. Verfahren nach einem der Ansprüche 2 bis 7, **dadurch gekennzeichnet, dass** nur ein Teil des Katalysatorstroms 2 für die Hydrocyanierung in Verfahrensschritt (d) verwendet wird und der restliche Teil des Katalysatorstroms 2 als By-pass-Strom 1' direkt in Verfahrensschritt (a') überführt wird.

10. Verfahren nach einem der Ansprüche 2 bis 8, **dadurch gekennzeichnet, dass** nur ein Teil des Katalysatorstroms 3 für die Hydrocyanierung in Verfahrensschritt (a) verwendet wird und der restliche Teil des Katalysatorstroms 3 als By-pass-Strom 2 direkt in Verfahrensschritt (a').

11. Verfahren nach einem der Ansprüche 1 bis 10, **dadurch gekennzeichnet, dass** in Verfahrensschritt (c) ein frischer und/oder reduktiv regenerierter Nickel(0)-Katalysator zugeführt wird.

12. Verfahren nach einem der Ansprüche 1 bis 11, **dadurch gekennzeichnet, dass** das Verfahren kontinuierlich durchgeführt wird.

## Claims

1. A process for preparing adiponitrile by hydrocyanating 1,3-butadiene over a catalyst, by,
- in a first process step, hydrocyanating 1,3-butadiene to 3-pentenenitrile over at least one nickel(0) catalyst and,
- in a second process step, hydrocyanating 3-pentenenitrile to adiponitrile over at least one nickel(0) catalyst with addition of at least one Lewis acid,
which comprises
transferring at least one of the Nickel (0) catalysts used in the particular process steps of
(a) hydrocyanating 1,3-butadiene over at least one nickel(0) catalyst, resulting in a hydrocyanation stream 1 which comprises 3-pentenenitrile, 2-pentenenitrile, 2-methyl-2-butenenitrile, C₉ nitriles, 2-methyl-3-butenenitrile, methylglutaronitrile, the at least one nickel(0) catalyst, unconverted 1,3-butadiene and residues of unconverted hydrogen cyanide,
(b) removing the at least one nickel(0) catalyst from the hydrocyanation stream 1 to obtain a catalyst stream 1 which comprises the at least one nickel(0) catalyst, and a hydrocyanation stream 2 which comprises 3-pentenenitrile, 2-pentenenitrile, 2-methyl-2-butenenitrile, C₉ nitriles and 2-methyl-3-butenenitrile,
(c) regenerating the at least one nickel(0) catalyst in the catalyst stream 1 by reductive nickel catalyst synthesis with addition of fresh ligand to obtain a catalyst stream 2,
(d) hydrocyanating 3-pentenenitrile over at least one nickel(0) catalyst and in the presence of at least one Lewis acid, the nickel(0) catalyst and the Lewis acid stemming at least partly from catalyst stream 2, resulting in a hydrocyanation stream 3 which comprises the at least one nickel(0) catalyst, adiponitrile and the at least one Lewis acid,
(e) removing the at least one nickel(0) catalyst from the hydrocyanation stream 3 by extraction with an organic solvent to obtain a catalyst stream 3 which comprises the at least one nickel(0) catalyst, and a product stream which comprises adiponitrile, and the catalyst stream 3 can be recycled at least partly into process step (a),
at least partly into the other process step in each case.

2. The process according to claim 1, **characterized by** the following process steps:
(a) hydrocyanating 1,3-butadiene over at least one nickel(0) catalyst, resulting in a hydrocyanation stream 1 which comprises 3-pentenenitrile, 2-pentenenitrile, 2-ethyl-2-butenenitrile, C₉ nitriles, methylglutaronitrile, 2-methyl-3-butenenitrile, the at least one nickel(0) catalyst, unconverted 1,3-butadiene and residues of unconverted hydrogen cyanide,
(a') isomerizing 2-methyl-3-butenenitrile which is present in the hydrocyanation stream 1 to 3-pentenenitrile over at least one nickel(0) catalyst to obtain a 2-methyl-3-butenenitrile-depleted and a 3-pentenenitrile-enriched isomerization stream 1,
(b) removing the at least one nickel(0) catalyst from the isomerization stream 1 to obtain a catalyst system 1 which comprises the at least one nickel(0) catalyst, and a hydrocyanation stream 2 which comprises 3-pentenenitrile, 2-pentenenitrile, 2-methyl-2-butenenitrile, C₉ nitriles and 2-methyl-3-butenenitrile,
(c) regenerating the at least one nickel(0) catalyst in the catalyst stream 1 by reductive nickel catalyst synthesis with addition of fresh ligand to obtain a catalyst stream 2,
(d) hydrocyanating 3-pentenenitrile over at least one nickel(0) catalyst and in the presence of at least one Lewis acid, the nickel(0) catalyst and the Lewis acid stemming at least partly from catalyst stream 2, resulting in a hydrocyanation stream 3 which comprises adiponitrile, the at least one nickel(0) catalyst and the at least one Lewis acid,
(e) removing the at least one nickel(0) catalyst from the hydrocyanation stream 2 by extraction with an organic solvent to obtain a catalyst stream 3 which comprises the at least one nickel(0) catalyst, and a product stream which comprises adiponitrile, and the catalyst stream 3 can be recycled at least partly into process step (a).

3. The process according to claim 2, wherein, in an additional process step (f), the hydrocyanation stream 2 is separated and a 3-pentenenitrile-rich stream 4 and a 2-methyl-3-butenenitrile-rich stream 5 are obtained.

4. The process according to claim 3, wherein the 3-pentenenitrile-rich stream 4 is conducted into process step (d).

5. The process according to claim 3 or 4, wherein the 2-methyl-3-butenenitrile-rich stream 5 is conducted into process step (a').

6. The process according to any of claims 1 to 5, wherein the process sequence begins with process step (d) in the first run when fresh Lewis acid and fresh nickel(0) catalyst and/or reductively regenerated nickel(0) catalyst are used.

7. The process according to any of claims 1 to 6, wherein 3-pentenenitrile hydrocyanated in process step (d) stems from the hydrocyanation stream 1 or the isomerization stream 1.

8. The process according to any of claims 1 to 7, wherein only a portion of the catalyst stream 2 is used for the hydrocyanation in process step (d) and the remaining portion of the catalyst stream 2 is transferred directly into process step (e) as a bypass stream 1.

9. The process according to any of claims 2 to 7, wherein only a portion of the catalyst stream 2 is used for the hydrocyanation in process step (d) and the remaining portion of the catalyst stream 2 is transferred directly into process step (a') as a bypass stream 2'.

10. The process according to any of claims 2 to 8, wherein only a portion of the catalyst stream 3 is used for the hydrocyanation in process step (a) and the remaining portion of the catalyst stream 3 is transferred directly into process step (a') as a bypass stream 2.

11. The process according to any of claims 1 to 10, wherein a fresh and/or reductively regenerated nickel(0) catalyst is fed into process step (c).

12. The process according to any of claims 1 to 11, which is carried out continuously.

## Revendications

1. Procédé de fabrication d'adiponitrile par hydrocyanation de 1,3-butadiène sur un catalyseur, selon lequel
- lors d'une première étape de procédé, le 1,3-butadiène est hydrocyané en 3-pentène-nitrile sur au moins un catalyseur de nickel (0) et
- lors d'une seconde étape de procédé, le 3-pentène-nitrile est hydrocyané en adiponitrile sur au moins un catalyseur de nickel (0) avec ajout d'au moins un acide de Lewis,
**caractérisé en ce qu'**au moins un des catalyseurs de nickel (0) utilisés dans les étapes de procédé respectives
(a) hydrocyanation de 1,3-butadiène sur au moins un catalyseur de nickel (0), un courant d'hydrocyanation 1 résultant, qui contient du 3-pentène-nitrile, du 2-pentène-nitrile, du 2-méthyl-2-butène-nitrile, des nitriles en C₉, du 2-méthyl-3-butène-nitrile, du méthylglutardinitrile, le ou les catalyseurs de nickel (0), du 1,3-butadiène non réagi et des résidus de cyanure d'hydrogène non réagi,
(b) séparation du ou des catalyseurs de nickel (0) du courant d'hydrocyanation 1 pour obtenir un courant de catalyseur 1, qui contient le ou les catalyseurs de nickel (0), et un courant d'hydrocyanation 2, qui contient du 3-pentène-nitrile, du 2-pentène-nitrile, du 2-méthyl-2-butène-nitrile, des nitriles en C₉ et du 2-méthyl-3-butène-nitrile,
(c) régénération du ou des catalyseurs de nickel (0) dans le courant de catalyseur 1 par synthèse par réduction du catalyseur de nickel avec ajout de ligand frais pour obtenir un courant de catalyseur 2,
(d) hydrocyanation de 3-pentène-nitrile sur au moins un catalyseur de nickel (0) et en présence d'au moins un acide de Lewis, le catalyseur de nickel (0) et l'acide de Lewis étant issus au moins en partie du courant de catalyseur 2 et un courant d'hydrocyanation 3 résultant, qui contient le ou les catalyseurs de nickel (0), de l'adiponitrile et le ou les acides de Lewis,
(e) séparation du ou des catalyseurs de nickel (0) du courant d'hydrocyanation 3 par extraction avec un solvant organique pour obtenir un courant de catalyseur 3, qui contient le ou les catalyseurs de nickel (0), et un courant de produit, qui contient de l'adiponitrile, le courant de catalyseur 3 pouvant être recyclé au moins en partie dans l'étape de procédé (a),
est transféré au moins en partie dans chacune des autres étapes de procédé.

2. Procédé selon la revendication 1, **caractérisé par** les étapes de procédé suivantes :
(a) hydrocyanation de 1,3-butadiène sur au moins un catalyseur de nickel (0), un courant d'hydrocyanation 1 résultant, qui contient du 3-pentène-nitrile, du 2-pentène-nitrile, du 2-méthyl-2-butène-nitrile, des nitriles en Cg, du méthylglutarnitrile, du 2-méthyl-3-butène-nitrile, le ou les catalyseurs de nickel (0), du 1,3-butadiène non réagi et des résidus de cyanure d'hydrogène non réagi,
(a') isomérisation de 2-méthyl-3-butène-nitrile, qui est contenu dans le courant d'hydrocyanation 1, en 3-pentène-nitrile sur au moins un catalyseur de nickel (0), un courant d'isomérisation 1 appauvri en 2-méthyl-3-butène-nitrile et enrichi en 3-pentène-nitrile étant obtenu,
(b) séparation du ou des catalyseurs de nickel (0) du courant d'isomérisation 1 pour obtenir un courant de catalyseur 1, qui contient le ou les catalyseurs de nickel (0), et un courant d'hydrocyanation 2, qui contient du 3-pentène-nitrile, du 2-pentène-nitrile, du 2-méthyl-2-butène-nitrile, des nitriles en C₉ et du 2-méthyl-3-butène-nitrile,
(c) régénération du ou des catalyseurs de nickel (0) dans le courant de catalyseur 1 par synthèse par réduction du catalyseur de nickel avec ajout de ligand frais pour obtenir un courant de catalyseur 2,
(d) hydrocyanation de 3-pentène-nitrile sur au moins un catalyseur de nickel (0) et en présence d'au moins un acide de Lewis, le catalyseur de nickel (0) et l'acide de Lewis étant issus au moins en partie du courant de catalyseur 2 et un courant d'hydrocyanation 3 résultant, qui contient de l'adiponitrile, le ou les catalyseurs de nickel (0) et le ou les acides de Lewis,
(e) séparation du ou des catalyseurs de nickel (0) du courant d'hydrocyanation 2 par extraction avec un solvant organique pour obtenir un courant de catalyseur 3, qui contient le ou les catalyseurs de nickel (0), et un courant de produit, qui contient de l'adiponitrile, le courant de catalyseur 3 pouvant être recyclé au moins en partie dans l'étape de procédé (a).

3. Procédé selon la revendication 2, **caractérisé en ce que** le courant d'hydrocyanation 2 est séparé lors d'une étape de procédé (f) supplémentaire et un courant 4 riche en 3-pentène-nitrile et un courant 5 riche en 2-méthyl-3-butène-nitrile sont obtenus.

4. Procédé selon la revendication 3, **caractérisé en ce que** le courant 4 riche en 3-pentène-nitrile est conduit dans l'étape de procédé (d).

5. Procédé selon la revendication 3 ou 4, **caractérisé en ce que** le courant 5 riche en 2-méthyl-3-butène-nitrile est conduit dans l'étape de procédé (a').

6. Procédé selon l'une quelconque des revendications 1 à 5, **caractérisé en ce que** le premier passage dans la séquence du procédé commence avec l'étape de procédé (d) avec ajout d'acide de Lewis frais et de catalyseur de nickel (0) frais et/ou de catalyseur de nickel (0) régénéré par réduction.

7. Procédé selon l'une quelconque des revendications 1 à 6, **caractérisé en ce que** le 3-pentène-nitrile hydrocyané à l'étape de procédé (d) est issu du courant d'hydrocyanation 1 ou du courant d'isomérisation 1.

8. Procédé selon l'une quelconque des revendications 1 à 7, **caractérisé en ce que** seule une partie du courant de catalyseur 2 est utilisée pour l'hydrocyanation à l'étape de procédé (d) et la partie restante du courant de catalyseur 2 est transférée directement sous la forme d'un courant de dérivation 1 dans l'étape de procédé (e).

9. Procédé selon l'une quelconque des revendications 2 à 7, **caractérisé en ce que** seule une partie du courant de catalyseur 2 est utilisée pour l'hydrocyanation à l'étape de procédé (d) et la partie restante du courant de catalyseur 2 est transférée directement sous la forme d'un courant de dérivation 1' dans l'étape de procédé (a').

10. Procédé selon l'une quelconque des revendications 2 à 8, **caractérisé en ce que** seule une partie du courant de catalyseur 3 est utilisée pour l'hydrocyanation à l'étape de procédé (a) et la partie restante du courant de catalyseur 3 est transférée directement sous la forme d'un courant de dérivation 2 dans l'étape de procédé (a').

11. Procédé selon l'une quelconque des revendications 1 à 10, **caractérisé en ce qu'**un catalyseur de nickel (0) frais et/ou régénéré par réduction est introduit dans l'étape de procédé (c).

12. Procédé selon l'une quelconque des revendications 1 à 11, **caractérisé en ce que** le procédé est réalisé en continu.
